# EUROPEAN PATENT SPECIFICATION

(11) **EP 2 751 085 B1**
(45) Date of publication and mention of the grant of the patent: **23.11.2016**
(21) Application number: 12827498.2
(22) Date of filing: 30.08.2012
(51) Int. Cl.: C07D 239/42, C07D 339/08, A01N 43/32, A01N 43/54, A01P 3/00

(54) **CO-CRYSTALS OF CYPRODINIL AND DITHIANON**
CO-KRISTALLE AUS CYPRODINIL UND DITHIANON
COCRISTAUX DE CYPRODINIL ET DE DITHIANON

(30) Priority: 30.08.2011 US 201161528776 P; 30.08.2011 EP 11179335; 13.03.2012 EP 12159171
(43) Date of publication of application: 09.07.2014
(73) Proprietor: BASF SE, 67056 Ludwigshafen am Rhein (DE)
(72) Inventor: SOWA, Christian, 67435 Neustadt (DE); GOLD, Randall Evan, 67283 Obrigheim (DE); CHIODO, Tiziana, 68159 Mannheim (DE); VOGEL, Ralf, 67117 Limburgerhof (DE)
(74) Representative: BASF IP Association
(86) International application number: PCT/IB2012/054452
(87) International publication number: WO 2013/030777

(56) References cited:
- WO-A1-2004/004461
- WO-A1-2009/047043
- WO-A1-2010/000612
- WO-A2-2007/036355
- WO-A2-2008/117060
- WO-A2-2010/038008
- STAHLY: "Diversity in Single- and Multiple-Component Crystals. The Search for and Prevalence of Polymorphs and Cocrystals", CRYSTAL GROWTH & DESIGN, ACS, WASHINGTON, DC, US, vol. 7, no. 6, 18 May 2007 (2007-05-18), pages 1007-1026, XP002483372, ISSN: 1528-7483, DOI: 10.1021/CG060838J [retrieved on 2007-05-18]

## Description

The present invention relates to novel co-crystals of cyprodinil and dithianon, to processes of their preparation and to the use of the novel co-crystals for the preparation of compositions for crop protection.

Fungicides are usually marketed as liquid or solid formulations which comprise one or more fungicidally active organic compounds and suitable formulation additives, more preferably two or more active organic compounds are used. For several reasons, formulation types are preferred, wherein the agriculturally active organic compounds are present in the solid state, examples including solid formulations such as dusts, powders or granules and liquid formulations such as suspension concentrates, i.e. liquid formulations containing solid particles of the active organic compound suspended in a liquid suspension medium.

For formulation purposes, the agriculturally active organic compounds should be a crystalline material having a sufficiently high melting point. Unfortunately, a large number of such organic compounds are amorphous material and/or have low melting points. Such compounds are difficult to formulate as suspension concentrates (SC) in a conventional manner, since the grinding apparatus will get stuck during grinding as a result of the tackiness of the active compound. Formulations of amorphous solid organic compounds are often instable with regard to phase-separation. For example, suspension concentrates of amorphous solid actives tend to become inhomogeneous by segregation of the active organic cyprodinils a result of particle aggregation or particle growth.

Co-crystals of organic compounds are multi-component crystals or crystalline material that consist of at least two different organic compounds which are usually solid at 25°C or at least a non-volatile oil (vapour pressure less than 1 mbar at 25°C). In the co-crystals at least two different organic compounds form together a crystalline material having a defined crystal structure, i. e. the at least two organic compounds have a defined relative spatial arrangement within the crystal structure, thereby forming a supra-molecular structure.

Dithianon is known as a fungicidally active organic compound described in GB-A 857 383.

In the literature several different melting points have been reported for dithianon crystalline modifications that are in the range of 180 to 240°C. Thermal analysis (Differential scanning calorimetry, DSC), has shown that it is very difficult if not impossible to give an exact melting point for any of the dithianon modifications. Instead of melting, it is found that the dithianon modifications undergo phase transformations to further modifications. Also decomposition of dithianon at high temperatures complicates the melting point determination. It is however clear that all of the dithianon modifications have melting points above 180°C.

Cyprodinil is known as a fungicidally active organic compound desribed in EP 0310 550 A1.

Co-crystals of cyprodinil with organic acids such as benzoic acid, maleic acid, fumaric acid, oxalic acid, pyrazine carboxylic acid, levulenic acid et al. are known from WO 2008/117060. Co-crystals of cyprodinil with various co-crystal forming small organic molecules from various chemical classes are known from WO 2010/038008. None of these compounds are known as fungicides and none of them are related to dithianon.

Two enantiotropically-related polymorphic forms of cyprodinil are known to exist (see WO 2008/117060), both of which exhibit characteristic, but different melting ranges 70 to 72°C for Form A and 74 to 76°C for Form B, respectively. It is also known that under practical storage conditions for formulations of agrochemicals, a solid state of cyprodinil may undergo transformation by re-crystallization between the two polymorphic forms leading to the generation of large and undesirable particles, which could for example, block spray nozzles during application of the product.

WO 2004/004461 describes mixtures of cyprodinil and dithianon which are synergistic. All biological experiments were conducted in a solution of the mixture in aceton and DMSO. After adding 1 % of an emulgator the solution was mixed with water to the desired concentration. Suspension concentrates were not described in this document.

Accordingly, it was an object of the present invention to provide a mixture of cyprodinil and dithianon in a form which permits the preparation of solid and liquid dispersions (i.e. suspension concentrates, suspo-emulsions and water-dispersible granules) which are convenient in the application and stable and/or do not undergo (re-)crystallization of the solids on formulation and/or storage.

This object has been achieved by the co-crystals of cyprodinil and dithianon as described below.

Therefore, the present invention relates to co-crystals comprising at least cyprodinil and dithianon.

The co-crystals can be distinguished from simple mixtures of crystalline cyprodinil and crystalline dithianon by standard analytical means used for the analysis of crystalline material, including powder X-ray diffractometry (PXRD), single crystal X-ray diffraction, IR and Ranman spectroscopy, solid state ¹³C-NMR (¹³C-CP/MAS: cross polarization - magic angle spinning) and thermochemical analysis such as thermogravimetry and differential thermal analysis (TG/DTA) and differential scanning calorimetry (DSC). The most important and most applicable analytical tool for the identification of a crystal modification is XRPD. Relative amounts of cyprodinil and dithianon can be determined e.g. by HPLC or by ¹H-NMR-spectroscopy.

The co-crystal of cyprodinil and dithianon shows a powder X-ray diffractogram at 20°C (Cu-Kα-radiation, 1.54060 Å). In particular, the co-crystal shows at least 4, preferably at least 6, in particular at least 8 and more preferably all of the following reflexes, given in the following table 1 as 2θ values or as lattice spacings d:

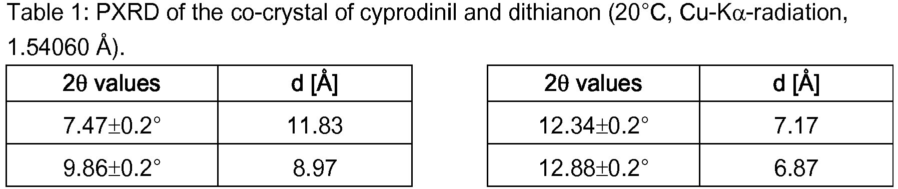

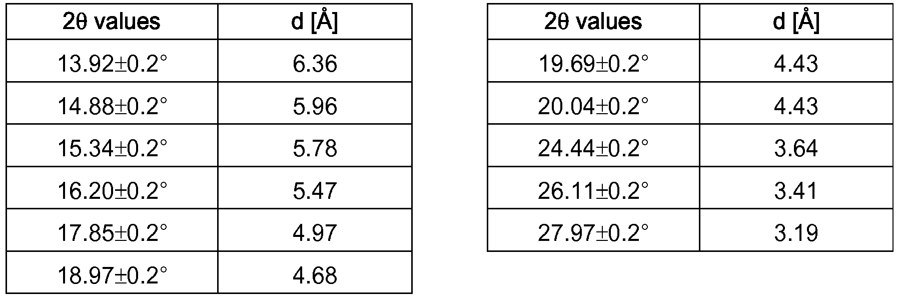

Thus, the present invention preferably relates to co-crystals of cyprodinil and dithianon, which, in a powder X-ray diffractogram at 20°C, show at least three, more preferably at least four, even more preferably at least six and in particular all of the following 2θ values [°]:
7.47±0.2; 9.86±0.2°; 12.34±0.2°; 12.88±0.2°; 13.92±0.2°; 14.88±0.2°; 15.34±0.2°; 16.20±0.2°; 17.85±0.2°; 18.97±0.2°; 19.69±0.2°; 20.04±0.2°; 24.44±0.2°; 26.11±0.2°; 27.97±0.2°.

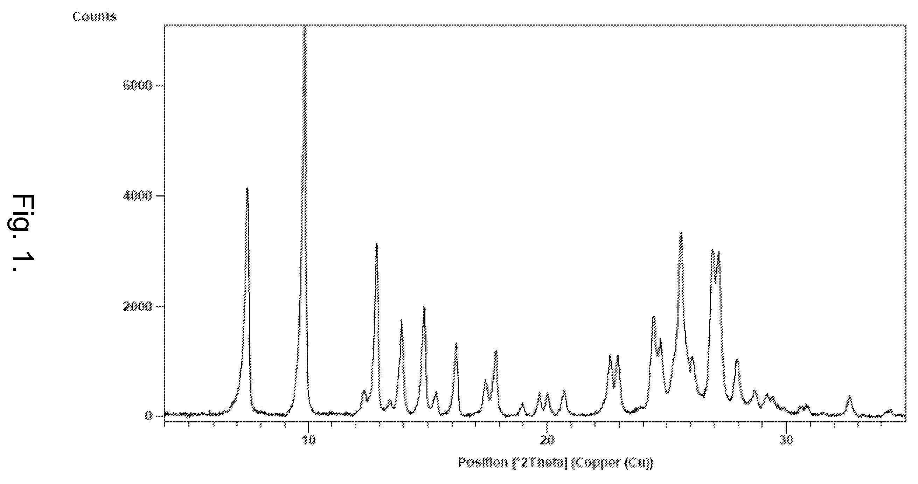

The powder diffraction pattern of the co-crystal can be found in FIG. 1.

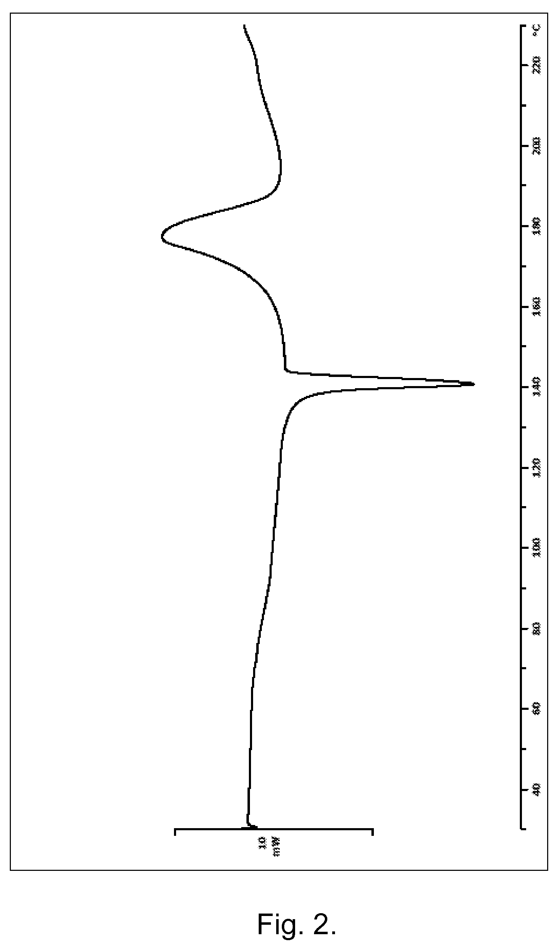

In DSC measurements (5°C/min, N₂-flow), the co-crystal of dithianon and cyprodinil shows an endothermic melting peak in between 125°C and 145°C, and from the onset temperature the melting point was determined to be 138°C (see FIG. 2).

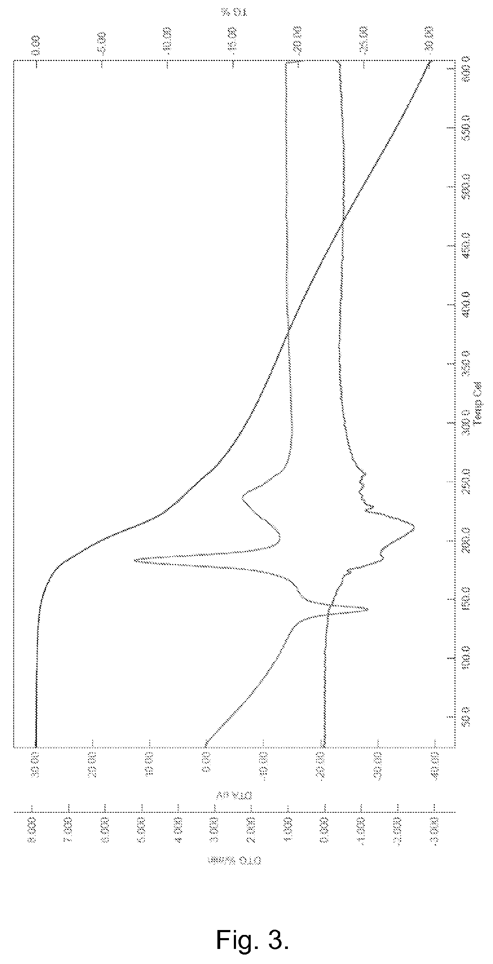

In a TG/DTA measurement (5°C/min, N₂-flow), no mass loss has been observed below the melting point (see FIG. 3).

In the co-crystals according to the present invention, the molar ratio of cyprodinil and dithianon is at least 0.5:1 and may vary from 0.5:1 to 2:1 and is preferably from 0.8:1 to 1.5:1 or 0.9:1 to 1.1:1. In particular, the molar ratio is about 1:1, however, deviations are possible, though they will generally not exceed 20 mol-% and preferably 10 mol-%.

The co-crystals according to the present invention have a defined crystal structure and have a reasonable high melting point which facilitates the incorporation of such complexes into solid or liquid formulations wherein the active material is present in the solid state. Moreover, the formulations of such co-crystals show increased stability, in particular in comparison with formulations containing a mixture of cyprodinil and dithianon as individual solid compounds.

The co-crystals of the present invention are suitable for the preparation of compositions for crop protection, in particular for the preparation of aqueous suspension concentrates.

Accordingly, the invention also provides a composition for crop protection, comprising co-crystals of cyprodinil and dithianon and at least one auxiliary.

The co-crystals of the present invention can be prepared by co-crystallizing dithianon and cyprodinil from a solution or slurry or from a melt containing cyprodinil and dithianon. The ratio of the co-crystals is not critical as the 1:1 co-crystal will form leaving the excess of the other components intact. Likewise, it is possible to prepare the co-crystals of the present invention by dry-mixing of about equimolar amounts of cyprodinil and dithianion to form a premix followed by dry-grinding the premix preferably at an elevated temperature, e.g. above 30°C, more preferably at a temperature of at least 40°C, in particular of at least 50°C, more preferably of at least 55°C, e.g. from >30°C to 110°C, preferably from 40°C to 100°C, in particular from 50°C to 90°C or from 55°C to 90°C. Cyprodinil may be solid at the grinding temperature. However, this is not necessary and it might be advantageous if the temperature is close to or above the melting point of cyprodinil.

Likewise, it is possible to prepare the co-crystal by mixing or grinding a mixture of dithianon and cyprodinil as dry compounds, with organic solvents or in aqueous or vegetable or mineral oil-based media. According to one embodiment, the organic solvents are selected from ketones, such as acetone, 2-butanone, cyclopentanone and cyclohexanone. According to a further embodiment, the organic solvents are alcohols such as methanol, ethanol, n-propanol, isopropanol or 1,2-propylenglycol. According to a further embodiment, the organic solvents are esters such as ethyl acetate. According to a further embodiment, the organic solvents are ethers such as diethyl ether, diisopropyl ether, methyl tert.-butyl ether (MTBE), dioxane, anisole and tetrahydrofuran (THF). In general, these organic solvents can be used in mixture with water.Likewise, it is possible to prepare the co-crystals by dissolving about equimolar amounts of cyprodinil and dithanion in a polar organic solvent followed by evaporating the solvent to form the co-crystals.

Likewise, it is possible to prepare the co-crystals by mixing about equimolar amounts of cyprodinil and dithanion with a liquid carrier to form a pre-mix, followed by grinding or agitating the premix or applying shear forces to the premix to form the co-crystals in the liquid carrier It is also possible to grind and agitate the premix and/or apply shear forces to the premix. Liquid carriers can be organic solvents, water, a mixture of water and organic solvent. The liquid carriers may further comprise auxiliaries such as dsiperging agents as defined herein.

In a preferred embodiment, the co-crystal of cyprodinil and dithianon is obtained from a slurry of cyprodinil and dithianon in an organic solvent or in a mixture of water and organic solvent. Consequently, this method comprises suspending cyprodinil and dithianon in an organic solvent or in a mixture of water and an organic solvent (slurry process).

Preferred organic solvents for the slurry process are those, which are at least partially water miscible, i.e. which have miscibility with water of at least 10 % v/v, more preferably at least 20 % v/v at room temperature, mixtures thereof and mixtures of said water miscible solvents with organic solvents that have miscibility with water of less than 10 % v/v at room temperature. Preferably the organic solvent comprises at least 80 % v/v, based on the total amount of organic solvent, of the at least one water miscible solvent.

Suitable solvents having a water miscibility of at least 10 % at room temperature include, but are not limited to:
1. C₁-C₄-Alkanols such as methanol, ethanol, n-propanol or isopropanol;
2. Amides, N-methylamides and N,N-dimethylamides of C₁-C₃-carboxylic acids such as formamide, dimethylformamide (DMF), acetamide and N,N-dimethylacetamide;
3. 5 or 6-membered lactames with a total of 7 carbon atoms such as pyrrolidone, N-methylpyrrolidone, N-ethylpyrrolidone, N-isopropylpyrrolidone, N-hydroxyethylpyrrolidone;
4. Dimethylsulfoxid and sulfolane;
5. Ketones with 3 to 6 carbon atoms such as acetone, 2-butanone, cyclopentanone and cyclohexanone;
6. Acetonitrile;
7. 5- or 6-membered lactones such as γ-butyrolactone;
8. Polyols and polyetherols such as glycol, glycerin, dimethoxyethan, ethylendiglycol, ethylenglycolmonomethylether, etc;
9. Cyclic carbonates having 3 to 5 carbon atoms including propylene carbonate and ethylene carbonate; and
10. Cyclic ethers such as tetrahydrofurane, dioxane and trioxane, dimethyl (poly)C₂-C₃-alkyleneglycol ethers such as dimethoxyethane, diethyleneglycoldimethylether, triethyleneglycoldimethylether, dipropyleneglycoldimethylether, low molecular weight polyethyleneglycoles and low molecular weight polypropyleneglycoles (MW ≤ 400).

More preference is given to organic solvents of the groups 1, 6, 8 and 9, and to their mixtures with water. In the mixtures with water the relative amount of organic solvent and water may vary from 10:1 to 1:10, in particular from 2:1 to 1:5.

The slurry process can by simply performed by suspending dithianon and cyprodinil in the organic solvent or solvent/water mixture. The relative amounts of dithianon, cyprodinil and solvent or solvent/water mixture will be chosen to obtain a suspension at the given temperature. Complete dissolution of dithianon and cyprodinil should be avoided. In particular dithianon and cyprodinil are suspended in an amount from 50 to 800 g, more preferably 100 to 600 g per litre of solvent or solvent/water mixture.

The relative molar amount of dithianon and cyprodinil may vary from 1:2 to 2:1, preferably from 1:1.5 to 1.5:1. If one of the components is in excess with regard to the stoichiometry of the co-crystal, a mixture of the co-crystal and the compound being in excess might be obtained, though a minor excess might remain dissolved in the mother liquor. For formulation purposes, the presence of an excess of cyprodinil or dithianon might be acceptable. In particular the presence of an excess of dithianon does not cause stability problems. For preparing the pure co-crystal, dithianon and cyprodinil will be used in a relative molar amount which is close to the stoichiometry of the complex to be formed and which usually will not deviate more than 50 mol-%, based on the stoichiometrically required amount.

The slurry process is usually performed at a temperature of at least 10°C, preferably at least 20°C and in particular at least 30°C, e.g. from 20 to 90°C, preferably from 30 to 85°C, in particular from 40 to 70°C.

Preferably, the slurry process in performed in the presence of a catalyst in form of a compatibility agent or solubilizer. A compatibility agent or solubilizer in the sense of the present invention is a substance that helps to transfer dithianon and/or cyprodinil over the phase, a process that seems essential for the co-crystal formation. Examples for such solubilizers or compatibility agents are solvents such as alcohols, and surfactants such as alcohol ethoxylates.

The time required for formation of the co-crystal by the slurry process depends on several paramters such as the temperature, the type of solvent and batch size. In any case, complete conversion is achieved after one week, however, the complete conversion will usually require not more than 24 h.

In another preferred embodiment of the invention, the co-crystal is prepared by applying shear forces to a liquid which contains suspended particles of cyprodinil and dithianon at a temperature of at least 10°C, more preferably at least 30°C, until the co-crystal has been formed (shear process).

In the liquid, dithianon and cyprodinil are present as particles, which are suspended in a liquid medium. Upon applying shear forces to the liquid at elevated temperatures the formation of the co-crystal takes place.

The main constituent of the liquid medium is water or an organic solvent, in which dithianon and cyprodinil is practically insoluble, i.e. the solubility at 25°C is less than 5 g/l, in particular less than 1 g/l. Suitable organic solvents include aliphatic hydrocarbons, mineral spirits, plant oils and plant oil esters. In a preferred embodiment, the liquid medium contains water or a mixture of water with up to 20 % v/v of a water miscible solvent, in particular a solvent of the group 1. or 9. as defined above, as main constituent. Apart from that, the liquid medium may also contain additives which are usually present in a liquid suspension concentrates.

The liquid medium may contain dithianon and cyprodinil in an amount from 5 to 70 % by weight, in particular from 10 to 60 % by weight and more preferably from 15 to 50 % by weight, based on the total weight of the liquid medium, cyprodinil and dithianon.

The liquid medium may contain dithianon and cyprodinil in a relative molar amount of dithianon and cyprodinil varying from 1:2 to 2:1, preferably from 1.5:1 to 1.5:1. If one of the components is in excess with regard to the stoichiometry of the co-crystal, a mixture of the co-crystal and the compound being in excess will be obtained. For formulation purposes, the presence of an excess of cyprodinil or dithianon might be acceptable. In particular the presence of an excess of dithianon does not cause stability problems. Likewise, the presence of an excess of cyprodinil does usually not cause stability problems. However, it is preferred, that a formulation does not contain both uncomplexed dithianon and uncomplexed cyprodinil in amounts of more than 20 % by weight each, nor in particular in amounts of more than 10 % by weight each, based on the amount of cyprodinil and thiophanate present in the form of the co-crystal, in order to avoid uncontrolled formation of the complex in the formulation. Therefore, the present invention relates in particular to formulations containing the co-crystal of the present invention, provided that, if both dithianon and cyprodinil are present in the formulation in non-complexed form, the amount of the cyprodinil does not exceed 20 % by weight, in particular 10 % by weight, based on the amount of complex in the formulation, and at the same time, the amount of dithianon does not exceed 20 % by weight, in particular 10 % by weight, based on the amount of complex in the formulation.

The liquid medium may include auxiliaries which are usually present in a liquid suspension concentrate. Suitable auxiliaries are described hereinafter and include surfactants, in particular anionic or non-ionic emulsifiers, wetting agents and dispersants usually employed in crop protection compositions, furthermore antifoam agents, anti-freeze agents, agents for adjusting the pH, stabilizers, anticaking agents, dyes and biocides (preservatives). Preferably, the liquid medium does not contain viscosity-modifying additives (thickeners). The amount of surfactants will generally be from 0.5 to 20% by weight, in particular from 1 to 15% by weight and particularly preferably from 1 to 10% by weight, based on the total weight of the liquid medium, cyprodinil, dithianon and auxiliaries. The amount of anti-freeze agents may be up to 10 % by weight, in particular up to 20 % by weight, e.g. from 0.5 to 20 % by weight, in particular from 1 to 10 % by weight, based on the total weight of the liquid medium, the cyprodinil and dithianon. Further additives, apart from anti-freeze agents and surfactants, may be present in amounts from 0 to 5 % by weight, based on the total weight of the liquid medium, cyprodinil, dithianon and auxiliaries.

The temperature required for formation of the co-crystal is generally at least 30°C, preferably at least 35°C and in particular at least 40°C, more preferably at least 50°C, especially at least 55°C, e.g. from 30 to 100°C, preferably from 35 to 100°C, in particular from 40 to 100°C, more preferably from 50 to 90°C and especially from 55 to 80°C.

The time required for formation of the co-crystal depends in a manner known per se on the type of shear process and the temperature and can be determined by the person skilled in the art in standard experiments. Shearing times in the range of e.g. from 30 min. to 48 hours have been found to be suitable, although a longer period of time is also conceivable. A shearing time of 1 to 24 hours is preferred.

Shear forces can be applied by suitable techniques, which are capable of providing sufficient shear to bring the particles of dithianon and cyprodinil into an intimate contact. Suitable techniques include grinding, crushing or milling, in particular by wet grinding or wet milling, including e.g. bead milling or by use of a colloid mill. Suitable shearing devices include in particular ball mills or bead mills, agitator ball mills, circulating mills (agitator ball mills with pin grinding system), disk mills, annular chamber mills, double cone mills, triple roll mills, batch mills, colloid mills, and media mills, such as sand mills. To dissipate the heat energy introduced during the grinding process, the grinding chambers are preferably fitted with cooling systems. Particularly suitable is the ball mill Drais Superflow DCP SF 12 from DRAISWERKE, INC.40 Whitney Road. Mahwah, NJ 07430 USA, a Drais Perl Mill PMC from DRAISWERKE, INC., the circulating mill system ZETA from Netzsch-Feinmahltechnik GmbH, the disk mill from Netzsch Feinmahltechnik GmbH, Selb, Germany, the bead mill Eiger Mini 50 from Eiger Machinery, Inc., 888 East Belvidere Rd., Grayslake, IL 60030 USA and the bead mill DYNO-Mill KDL from WA Bachofen AG, Switzerland. However, other homogenizers might also be suitable, including high shear stirrers, Ultra-Turrax apparatus, static mixers, e.g. systems having mixing nozzles and other homogenizers such as colloid mills.

In a preferred embodiment of the invention, shear is applied by bead milling. In particular, bead sizes in the range of from 0.05 to 5 mm, more particularly from 0.2 to 2.5 mm, and most particularly from 0.5 to 1.5 mm have been found to be suitable. In general, bead loadings in the range of from 40 to 9%, particularly from 70 to 97 %, and more particularly from 65 to 95% may be used.

After having applied sufficient shear forces a suspension of the co-crystal, optionally in admixture with excess dithianon or cyprodinil, is obtained, wherein 90 % by weight of the suspended particles have the particle size of not more than 30 µm, preferably not more than 20 µm, in particular not more than 10 µm especially not more than 5 µm, as determined by dynamic light scattering.

The obtained liquid suspension of the co-crystal can, after, or in particular before a formulation with auxiliaries, be converted by customary drying methods, in particular by spray-drying or freeze-drying, into powder compositions. Before or during drying, a drying or spray auxiliary may be added. Suitable drying or spray auxiliaries for drying aqueous dispersions are known. These include protective colloids, such as polyvinyl alcohol, in particular polyvinyl alcohol having a degree of hydrolysis of > 70%, carboxylated polyvinyl alcohol, phenolsulfonic acid/formaldehyde condensates, phenolsulfonic acid/urea/formaldehyde condensates, naphthalenesulfonic acid/formaldehyde condensates, naphthalenesulfonic acid/formaldehyde/urea condensates, polyvinylpyrrolidone, copolymers of maleic acid (or maleic anhydride) and vinylaromatics such as styrene and ethoxylated derivatives thereof, copolymers of maleic acid or maleic anhydride with C₂-C₁₀-olefins, such as diisobutene, and ethoxylated derivatives thereof, cationic polymers, for example homo- and copolymers of N-alkyl-N-vinylimidazolinium compounds with N-vinyl lactams and the like, and also inorganic anti-blocking agents (sometimes also termed as anti-caking agents), such as silicic acid, in particular pyrogenic silica, alumina, calcium carbonate and the like. The drying auxiliaries are usually employed in an amount of from 0.1 to 20% by weight, based on the weight of the active compound particles in the liquid pesticide composition of the present invention.

As already mentioned above, the co-crystal as defined herein are suitable for preparing crop protection compositions and in particular for preparing aqueous suspension concentrates. Accordingly, the invention also provides a composition for crop protection, comprising a co-crystal as defined herein, if appropriate a liquid phase and also, if appropriate, customary, generally auxiliaries such as solid carriers.

Thus, the invention also relates to agrochemical compositions comprising an auxiliary and at least co-crystals comprising at least dithianon and cyprodinil according to the invention.

According to another embodiment, these agrochemical compositions comprise beside the co-crystals and auxiliaries dithianon and/or cyprodinil (meaingin additional amounts of cyprodinil and/or dithianon e.g. in case that dithianon or cyprodinil is present in more than equimolar amounts).

According to a further embodiment, these agrochemical compositions additionally comprise at least one further active substance as deined herein. Preferably, the further active substances are selected from the classes A) to O) as defined below.

An agrochemical composition comprises a fungicidally effective amount of the coi-crystals according to the invention. The term "effective amount" denotes an amount of the composition or of the co-crystals, which is sufficient for controlling harmful fungi on cultivated plants or in the protection of materials and which does not result in a substantial damage to the treated plants. Such an amount can vary in a broad range and is dependent on various factors, such as the fungal species to be controlled, the treated cultivated plant or material, the climatic conditions and the specific co-crystals used.

The co-crystals can be converted into customary types of agrochemical compositions, emulsions, suspensions, dusts, powders, pastes, granules, pressings, capsules, and mixtures thereof. Examples for composition types are suspensions (e.g. SC, OD, FS), capsules (e.g. CS, ZC), pastes, pastilles, wettable powders or dusts (e.g. WP, SP, WS, DP, DS), pressings (e.g. BR, TB, DT), granules (e.g. WG, SG, GR, FG, GG, MG), insecticidal articles (e.g. LN), as well as gel formulations for the treatment of plant propagation materials such as seeds (e.g. GF). These and further compositions types are defined in the "Catalogue of pesticide formulation types and international coding system", Technical Monograph No. 2, 6th Ed. May 2008, CropLife International.

The compositions are prepared in a known manner, such as described by Mollet and Grubemann, Formulation technology, Wiley VCH, Weinheim, 2001; or Knowles, New developments in crop protection product formulation, Agrow Reports DS243, T&F Informa, London, 2005.

Suitable auxiliaries are solvents, liquid carriers, solid carriers or fillers, surfactants, dispersants, emulsifiers, wetters, adjuvants, solubilizers, penetration enhancers, protective colloids, adhesion agents, thickeners, humectants, repellents, attractants, feeding stimulants, compatibilizers, bactericides, anti-freezing agents, anti-foaming agents, colorants, tackifiers and binders.

Suitable solvents and liquid carriers are water and organic solvents, such as mineral oil fractions of medium to high boiling point, e.g. kerosene, diesel oil; oils of vegetable or animal origin; aliphatic, cyclic and aromatic hydrocarbons, e. g. toluene, paraffin, tetrahydronaphthalene, alkylated naphthalenes; alcohols, e.g. ethanol, propanol, butanol, benzylalcohol, cyclohexanol; glycols; DMSO; ketones, e.g. cyclohexanone; esters, e.g. lactates, carbonates, fatty acid esters, gamma-butyrolactone; fatty acids; phosphonates; amines; amides, e.g. N-methylpyrrolidone, fatty acid dimethylamides; and mixtures thereof.

Suitable solid carriers or fillers are mineral earths, e.g. silicates, silica gels, talc, kaolins, limestone, lime, chalk, clays, dolomite, diatomaceous earth, bentonite, calcium sulfate, magnesium sulfate, magnesium oxide; polysaccharides, e.g. cellulose, starch; fertilizers, e.g. ammonium sulfate, ammonium phosphate, ammonium nitrate, ureas; products of vegetable origin, e.g. cereal meal, tree bark meal, wood meal, nutshell meal, and mixtures thereof.

Suitable surfactants are surface-active compounds, such as anionic, cationic, nonionic and amphoteric surfactants, block polymers, polyelectrolytes, and mixtures thereof. Such surfactants can be used as emusifier, dispersant, solubilizer, wetter, penetration enhancer, protective colloid, or adjuvant. Examples of surfactants are listed in McCutcheon' s, Vol.1: Emulsifiers & Detergents, McCutcheon' s Directories, Glen Rock, USA, 2008 (International Ed. or North American Ed.).

Suitable anionic surfactants are alkali, alkaline earth or ammonium salts of sulfonates, sulfates, phosphates, carboxylates, and mixtures thereof. Examples of sulfonates are alkylarylsulfonates, diphenylsulfonates, alpha-olefin sulfonates, lignine sulfonates, sulfonates of fatty acids and oils, sulfonates of ethoxylated alkylphenols, sulfonates of alkoxylated arylphenols, sulfonates of condensed naphthalenes, sulfonates of dodecyl- and tridecylbenzenes, sulfonates of naphthalenes and alkylnaphthalenes, sulfosuccinates or sulfosuccinamates. Examples of sulfates are sulfates of fatty acids and oils, of ethoxylated alkylphenols, of alcohols, of ethoxylated alcohols, or of fatty acid esters. Examples of phosphates are phosphate esters. Examples of carboxylates are alkyl carboxylates, and carboxylated alcohol or alkylphenol ethoxylates.

Suitable nonionic surfactants are alkoxylates, N-subsituted fatty acid amides, amine oxides, esters, sugar-based surfactants, polymeric surfactants, and mixtures thereof. Examples of alkoxylates are compounds such as alcohols, alkylphenols, amines, amides, arylphenols, fatty acids or fatty acid esters which have been alkoxylated with 1 to 50 equivalents. Ethylene oxide and/or propylene oxide may be employed for the alkoxylation, preferably ethylene oxide. Examples of N-subsititued fatty acid amides are fatty acid glucamides or fatty acid alkanolamides. Examples of esters are fatty acid esters, glycerol esters or monoglycerides. Examples of sugar-based surfactants are sorbitans, ethoxylated sorbitans, sucrose and glucose esters or alkylpolyglucosides. Examples of polymeric surfactants are home- or copolymers of vinylpyrrolidone, vinylalcohols, or vinylacetate.

Suitable cationic surfactants are quaternary surfactants, for example quaternary ammonium compounds with one or two hydrophobic groups, or salts of long-chain primary amines. Suitable amphoteric surfactants are alkylbetains and imidazolines. Suitable block polymers are block polymers of the A-B or A-B-A type comprising blocks of polyethylene oxide and polypropylene oxide, or of the A-B-C type comprising alkanol, polyethylene oxide and polypropylene oxide. Suitable polyelectrolytes are polyacids or polybases. Examples of polyacids are alkali salts of polyacrylic acid or polyacid comb polymers. Examples of polybases are polyvinylamines or polyethyleneamines.

Suitable adjuvants are compounds, which have a neglectable or even no pesticidal activity themselves, and which improve the biological performance of the compound I on the target. Examples are surfactants, mineral or vegetable oils, and other auxilaries. Further examples are listed by Knowles, Adjuvants and additives, Agrow Reports DS256, T&F Informa UK, 2006, chapter 5.

Suitable thickeners are polysaccharides (e.g. xanthan gum, carboxymethylcellulose), anorganic clays (organically modified or unmodified), polycarboxylates, and silicates.

Suitable bactericides are bronopol and isothiazolinone derivatives such as alkylisothiazolinones and benzisothiazolinones.

Suitable anti-freezing agents are ethylene glycol, propylene glycol, urea and glycerin.

Suitable anti-foaming agents are silicones, long chain alcohols, and salts of fatty acids.

Suitable colorants (e.g. in red, blue, or green) are pigments of low water solubility and water-soluble dyes. Examples are inorganic colorants (e.g. iron oxide, titan oxide, iron hexacyanoferrate) and organic colorants (e.g. alizarin-, azo- and phthalocyanine colorants).

Suitable tackifiers or binders are polyvinylpyrrolidons, polyvinylacetates, polyvinyl alcohols, polyacrylates, biological or synthetic waxes, and cellulose ethers. The invention relates in particular to compositions for crop protection in the form of suspension concentrate, in particular an aqueous suspension concentrate (SC). Such suspension concentrates comprise the co-crystal in a finely divided particulate form, where the particles of the co-crystal are suspended in an liquid medium, preferably in an aqueous medium. The size of the active compound particles, i.e. the size which is not exceeded by 90% by weight of the active compound particles, is typically not more than 30 µm, preferably not more than 20 µm, in particular not more than 10 µm, especially not more than 5 µm, as determined by dynamic light scattering. Advantageously, at least 40% by weight and in particular at least 60% by weight of the particles in the SCs according to the invention have diameters below 2 µm.

Suspension concentrates, in particular aqueous suspension concentrates can be prepared by suspending the co-crystals in a suitable liquid carrier, which may contain conventional formulation additives as described hereinafter. However, it is preferred to prepare the suspension concentrate by the shear process as described herein, i.e. by applying shear forces to a liquid which contains suspended particles of cyprodinil and cyprodinil and optionally further additives at a temperature of at least 30 °C until the co-crystal has been formed.

Likewise, it is possible to prepare an aqueous suspension concentrate by adding cyprodinil into water which contains one or more dispersing agents and optionally other auxiliaries to form a premix A, followed by mixing the premix A with dithianon under stirring at a temperature from 20°C to 80°C or by adding dithianon into water which contains one or more dispersing agents and optionally other auxiliaries to form a premix B and mixing the premixes A and B together under stirring at a temperature from 20°C to 80°C to form a suspension of the co-crystals. Finally the so obtained suspension can be milled to a particle size distribution of about 60 % < 2 µ m.

Likewise, it is possible to prepare an aqueous suspension concentrate by adding dithianon into water which contains one or more dispersing agents and optionally other auxiliaries to form a premix B, followed by mixing the premix B with cyprodinil under stirring at a temperature from 20°C to 80°C to form a suspension of co-crystals, and finally milling the so obtained suspension to a particle size distribution of about 60 % < 2 µ m.

Preferably, the stirring step in these processes is at a temperature from 40 to 60°C. Preferably, the milling step in these processes is at a temperature from 20 to 30°C. In addition to the co-crystals, suspension concentrates typically comprise surfactants, and also, if appropriate, antifoam agents, thickeners, antifreeze agents, stabilizers (biocides), agents for adjusting the pH and anticaking agents.

In such SCs, the amount of active compounds, i.e. the total amount of the co-crystals according to the invention and, if appropriate, further active compounds, is usually in the range from 10 to 70% by weight, in particular in the range from 15 to 50% by weight, based on the total weight of the suspension concentrate.

Examples for composition types and their preparation are:
i) Suspensions (SC, OD, FS)
   In an agitated ball mill, 20-60 wt% of the co-crystals are comminuted with addition of 2-10 wt% dispersants and wetting agents (e.g. sodium lignosulfonate and alcohol ethoxylate), 0.1-2 wt% thickener (e.g. xanthan gum) and water ad 100 wt% to give a fine active substance suspension. Dilution with water gives a stable suspension of the active substance. For FS type composition up to 40 wt% binder (e.g. polyvinylalcohol) is added.
ii) Water-dispersible granules and water-soluble granules (WG, SG)
   50-80 wt% of the co-crystals are ground finely with addition of dispersants and wetting agents (e.g. sodium lignosulfonate and alcohol ethoxylate) ad 100 wt% and prepared as water-dispersible or water-soluble granules by means of technical appliances (e. g. extrusion, spray tower, fluidized bed). Dilution with water gives a stable dispersion or solution of the active substance.
iii) Water-dispersible powders and water-soluble powders (WP, SP, WS)
   50-80 wt% of the co-crystals are ground in a rotor-stator mill with addition of 1-5 wt% dispersants (e.g. sodium lignosulfonate), 1-3 wt% wetting agents (e.g. alcohol ethoxylate) and solid carrier (e.g. silica gel) ad 100 wt%. Dilution with water gives a stable dispersion or solution of the active substance.
iv) Gel (GW, GF)
   In an agitated ball mill, 5-25 wt% of the co-crystals are comminuted with addition of 3-10 wt% dispersants (e.g. sodium lignosulfonate), 1-5 wt% thickener (e.g. carboxymethylcellulose) and water ad 100 wt% to give a fine suspension of the active substance. Dilution with water gives a stable suspension of the active substance.
v)
vi) Dustable powders (DP, DS)
   1-10 wt% of the co-crystals are ground finely and mixed intimately with solid carrier (e.g. finely divided kaolin) ad 100 wt%.
vii) Granules (GR, FG)
   0.5-30 wt% of the co-crystals is ground finely and associated with solid carrier (e.g. silicate) ad 100 wt%. Granulation is achieved by extrusion, spray-drying or fluidized bed.

The agrochemical compositions generally comprise between 0.01 and 95%, preferably between 0.1 and 90%, and in particular between 0.5 and 75%, by weight of active substances. The active substances are employed in a purity of from 90% to 100%, preferably from 95% to 100% (according to NMR spectrum).

Suspoemulsions (SE), flowable concentrates (FS), powders for dry treatment (DS), water-dispersible powders for slurry treatment (WS), water-soluble powders (SS), emulsions (ES) and gels (GF) are usually employed for the purposes of treatment of plant propagation materials, particularly seeds. The compositions in question give, after two-to-tenfold dilution, active substance concentrations of from 0.01 to 60% by weight, preferably from 0.1 to 40%, in the ready-to-use preparations. Application can be carried out before or during sowing. Methods for applying the co-crystals and compositions thereof, respectively, on to plant propagation material, especially seeds include dressing, coating, pelleting, dusting, soaking and in-furrow application methods of the propagation material. Preferably, the co-crystals or the compositions thereof, respectively, are applied on to the plant propagation material by a method such that germination is not induced, e. g. by seed dressing, pelleting, coating and dusting.

When employed in plant protection, the amounts of active substances applied are, depending on the kind of effect desired, from 0.001 to 2 kg per ha, preferably from 0.005 to 2 kg per ha, more preferably from 0.05 to 0.9 kg per ha, and in particular from 0.1 to 0.75 kg per ha.

In treatment of plant propagation materials such as seeds, e. g. by dusting, coating or drenching seed, amounts of active substances of from 0.1 to 1000 g, preferably from 1 to 1000 g, more preferably from 1 to 100 g and most preferably from 5 to 100 g, per 100 kilogram of plant propagation material (preferably seeds) are generally required.

When used in the protection of materials or stored products, the amount of active substances applied depends on the kind of application area and on the desired effect. Amounts customarily applied in the protection of materials are 0.001 g to 2 kg, preferably 0.005 g to 1 kg, of active substance per cubic meter of treated material.

Various types of oils, wetters, adjuvants, fertilizer, or micronutrients, and further pesticides (e.g. herbicides, insecticides, fungicides, growth regulators, safeners) may be added to the active substances or the compositions comprising them as premix or, if appropriate not until immediately prior to use (tank mix). These agents can be admixed with the compositions according to the invention in a weight ratio of 1:100 to 100:1, preferably 1:10 to 10:1.

The user applies the composition according to the invention usually from a predosage device, a knapsack sprayer, a spray tank, a spray plane, or an irrigation system. Usually, the agrochemical composition is made up with water, buffer, and/or further auxiliaries to the desired application concentration and the ready-to-use spray liquor or the agrochemical composition according to the invention is thus obtained. Usually, 20 to 2000 liters, preferably 50 to 400 liters, of the ready-to-use spray liquor are applied per hectare of agricultural useful area.

According to one embodiment, individual components of the composition according to the invention such as parts of a kit or parts of a binary or ternary mixture may be mixed by the user himself in a spray tank and further auxiliaries may be added, if appropriate.

Mixing the co-crystals or the compositions comprising them in the use form as fungicides with other fungicides results in many cases in an expansion of the fungicidal spectrum of activity being obtained or in a prevention of fungicide resistance development. Furthermore, in many cases, synergistic effects are obtained.

The following list of active substances, in conjunction with which the co-crystals can be used, is intended to illustrate the possible combinations but does not limit them:
A) Respiration inhibitors
   - Inhibitors of complex III at Qₒ site (e.g. strobilurins): azoxystrobin, coumethoxystrobin, coumoxystrobin, dimoxystrobin, enestroburin, fenaminstrobin, fenoxystrobin/flufenoxystrobin, fluoxastrobin, kresoxim-methyl, metominostrobin, orysastrobin, picoxystrobin, pyraclostrobin, pyrametostrobin, pyraoxystrobin, trifloxystrobin, 2-[2-(2,5-dimethyl-phenoxymethyl)-phenyl]-3-methoxy-acrylic acid methyl ester and 2-(2-(3-(2,6-dichlorophenyl)-1-methyl-allylideneaminooxymethyl)-phenyl)-2-methoxyimino-N-methyl-acetamide, pyribencarb, triclopyricarb/chlorodin-carb, famoxadone, fenamidone;
   - inhibitors of complex III at Qᵢ site: cyazofamid, amisulbrom, [(3S,6S,7R,8R)-8-benzyl-3-[(3-acetoxy-4-methoxy-pyridine-2-carbonyl)amino]-6-methyl-4,9-dioxo-1,5-dioxonan-7-yl]2-methylpropanoate, [(3S,6S,7R,8R)-8-benzyl-3-[[3-(acetoxymethoxy)-4-methoxy-pyridine-2-carbonyl]amino]-6-methyl-4,9-dioxo-1,5-dioxonan-7-yl] 2-methylpropanoate, [(3S,6S,7R,8R)-8-benzyl-3-[(3-isobutoxycarbonyloxy-4-methoxy-pyridine-2-carbonyl)amino]-6-methyl-4,9-dioxo-1,5-dioxonan-7-yl] 2-methylpropanoate, [(3S,6S,7R,8R)-8-benzyl-3-[[3-(1,3-benzodioxol-5-ylmethoxy)-4-methoxypyridine-2-carbonyl]amino]-6-methyl-4,9-dioxo-1,5-dioxonan-7-yl] 2-methylpropanoate;
   - inhibitors of complex II (e. g. carboxamides): benodanil, bixafen, boscalid, carboxin, fenfuram, fluopyram, flutolanil, fluxapyroxad, furametpyr, isopyrazam, mepronil, oxycarboxin, penflufen, penthiopyrad, sedaxane, tecloftalam, thifluzamide, N-(4'-trifluoromethylthiobiphenyl-2-yl)-3-difluoromethyl-1-methyl-1H-pyrazole-4-carboxamide, N-(2-(1,3,3-trimethyl-butyl)-phenyl)-1,3-dimethyl-5-fluoro-1H-pyrazole-4-carboxamide, N-[9-(dichloromethylene)-1,2,3,4-tetrahydro-1,4-methanonaphthalen-5-yl]-3-(difluoromethyl)-1-methyl-1 H-pyrazole-4-carboxamide;
   - other respiration inhibitors (e.g. complex I, uncouplers): diflumetorim, (5,8-difluoroquinazolin-4-yl)-{2-[2-fluoro-4-(4-trifluoromethylpyridin-2-yloxy)-phenyl]-ethyl}-amine; nitrophenyl derivates: binapacryl, dinobuton, dinocap, fluazinam; ferimzone; organometal compounds: fentin salts, such as fentin-acetate, fentin chloride or fentin hydroxide; ametoctradin; and silthiofam;
B) Sterol biosynthesis inhibitors (SBI fungicides)
   - C14 demethylase inhibitors (DMI fungicides): triazoles: azaconazole, bitertanol, bromuconazole, cyproconazole, difenoconazole, diniconazole, diniconazole-M, epoxiconazole, fenbuconazole, fluquinconazole, flusilazole, flutriafol, hexaconazole, imibenconazole, ipconazole, metconazole, myclobutanil, oxpoconazole, paclobutrazole, penconazole, propiconazole, prothioconazole, simeconazole, tebuconazole, tetraconazole, triadimefon, triadimenol, triticonazole, uniconazole; imidazoles: imazalil, pefurazoate, prochloraz, triflumizol; pyrimidines, pyridines and piperazines: fenarimol, nuarimol, pyrifenox, triforine;
   - Delta14-reductase inhibitors: aldimorph, dodemorph, dodemorph-acetate, fenpropimorph, tridemorph, fenpropidin, piperalin, spiroxamine;
   - Inhibitors of 3-keto reductase: fenhexamid;
C) Nucleic acid synthesis inhibitors
   - phenylamides or acyl amino acid fungicides: benalaxyl, benalaxyl-M, kiralaxyl, metalaxyl, metalaxyl-M (mefenoxam), ofurace, oxadixyl;
   - others: hymexazole, octhilinone, oxolinic acid, bupirimate, 5-fluorocytosine, 5-fluoro-2-(p-tolylmethoxy)pyrimidin-4-amine, 5-fluoro-2-(4-fluorophenylmethoxy)pyrimidin-4-amine;
D) Inhibitors of cell division and cytoskeleton
   - tubulin inhibitors, such as benzimidazoles, thiophanates: benomyl, carbendazim, fuberidazole, thiabendazole, thiophanate-methyl; triazolopyrimidines: 5-chloro-7-(4-methylpiperidin-1-yl)-6-(2,4,6-trifluorophenyl)-[1,2,4]triazolo[1,5-a]pyrimidine
   - other cell division inhibitors: diethofencarb, ethaboxam, pencycuron, fluopicolide, zoxamide, metrafenone, pyriofenone;
E) Inhibitors of amino acid and protein synthesis
   - methionine synthesis inhibitors (anilino-pyrimidines): mepanipyrim, pyrimethanil;
   - protein synthesis inhibitors: blasticidin-S, kasugamycin, kasugamycin hydrochloride-hydrate, mildiomycin, streptomycin, oxytetracyclin, polyoxine, validamycin A;
F) Signal transduction inhibitors
   - MAP / histidine kinase inhibitors: fluoroimid, iprodione, procymidone, vinclozolin, fenpiclonil, fludioxonil;
   - G protein inhibitors: quinoxyfen;
G) Lipid and membrane synthesis inhibitors
   - Phospholipid biosynthesis inhibitors: edifenphos, iprobenfos, pyrazophos, isoprothiolane;
   - lipid peroxidation: dicloran, quintozene, tecnazene, tolclofos-methyl, biphenyl, chloroneb, etridiazole;
   - phospholipid biosynthesis and cell wall deposition: dimethomorph, flumorph, mandipropamid, pyrimorph, benthiavalicarb, iprovalicarb, valifenalate and N-(1-(1-(4-cyano-phenyl)ethanesulfonyl)-but-2-yl) carbamic acid-(4-fluorophenyl) ester;
   - compounds affecting cell membrane permeability and fatty acides: propamocarb, propamocarb-hydrochlorid
H) Inhibitors with Multi Site Action
   - inorganic active substances: Bordeaux mixture, copper acetate, copper hydroxide, copper oxychloride, basic copper sulfate, sulfur;
   - thio- and dithiocarbamates: ferbam, mancozeb, maneb, metam, metiram, propineb, thiram, zineb, ziram;
   - organochlorine compounds (e.g. phthalimides, sulfamides, chloronitriles): anilazine, chlorothalonil, captafol, captan, folpet, dichlofluanid, dichlorophen, flusulfamide, hexachlorobenzene, pentachlorphenole and its salts, phthalide, tolylfluanid, N-(4-chloro-2-nitro-phenyl)-N-ethyl-4-methyl-benzenesulfonamide;
   - guanidines and others: guanidine, dodine, dodine free base, guazatine, guazatine-acetate, iminoctadine, iminoctadine-triacetate, iminoctadine-tris(albesilate);
I) Cell wall synthesis inhibitors
   - inhibitors of glucan synthesis: validamycin, polyoxin B; melanin synthesis inhibitors: pyroquilon, tricyclazole, carpropamid, dicyclomet, fenoxanil;
J) Plant defence inducers
   - acibenzolar-S-methyl, probenazole, isotianil, tiadinil, prohexadione-calcium; phosphonates: fosetyl, fosetyl-aluminum, phosphorous acid and its salts;
K) Unknown mode of action
   - bronopol, chinomethionat, cyflufenamid, cymoxanil, dazomet, debacarb, diclomezine, difenzoquat, difenzoquat-methylsulfate, diphenylamin, fenpyrazamine, flumetover, flusulfamide, flutianil, methasulfocarb, nitrapyrin, nitrothal-isopropyl, oxin-copper, proquinazid, tebufloquin, tecloftalam, triazoxide, 2-butoxy-6-iodo-3-propylchromen-4-one, N-(cyclopropylmethoxyimino-(6-difluoro-methoxy-2,3-difluoro-phenyl)-methyl)-2-phenyl acetamide, N'-(4-(4-chloro-3-trifluoromethyl-phenoxy)-2,5-dimethyl-phenyl)-N-ethyl-N-methyl formamidine, N'-(4-(4-fluoro-3-trifluoro-methyl-phenoxy)-2,5-dimethyl-phenyl)-N-ethyl-N-methyl formamidine, N'-(2-methyl-5-trifluoromethyl-4-(3-trimethylsilanyl-propoxy)-phenyl)-N-ethyl-N-methyl formamidine, N'-(5-difluoromethyl-2-methyl-4-(3-trimethylsilanyl-propoxy)-phenyl)-N-ethyl-N-methyl formamidine, 2-{1-[2-(5-methyl-3-trifluoromethyl-pyrazole-1-yl)-acetyl]-piperidin-4-yl}-thiazole-4-carboxylic acid methyl-(1,2,3,4-tetrahydro-naphthalen-1-yl)-amide, 2-{1-[2-(5-methyl-3-trifluoromethyl-pyrazole-1-yl)-acetyl]-piperidin-4-yl}-thiazole-4-carboxylic acid methyl-(R)-1,2,3,4-tetrahydro-naphthalen-1-yl-amide, 1-[4-[4-[5-(2,6-difluorophenyl)-4,5-dihydro-3-isoxazolyl]-2-thiazolyl]-1-piperidinyl]-2-[5-methyl-3-(trifluoromethyl)-1 H-pyrazol-1-yl]ethanone, methoxy-acetic acid 6-tert-butyl-8-fluoro-2,3-dimethyl-quinolin-4-yl ester, *N*-Methyl-2-{1-[(5-methyl-3-trifluoromethyl-1H-pyrazol-1-yl)-acetyl]-piperidin-4-yl}-*N*-[(1R)-1,2,3,4-tetrahydro-naphthalen-1-yl]-4-thiazolecarboxamide, 3-[5-(4-methylphenyl)-2,3-dimethyl-isoxazolidin-3-yl]-pyridine, 3-[5-(4-chloro-phenyl)-2,3-dimethyl-isoxazolidin-3-yl]-pyridine (pyrisoxazole), N-(6-methoxy-pyridin-3-yl) cyclopropanecarboxylic acid amide, 5-chloro-1-(4,6-dimethoxy-pyrimidin-2-yl)-2-methyl-1H-benzoimidazole, 2-(4-chloro-phenyl)-N-[4-(3,4-dimethoxy-phenyl)-isoxazol-5-yl]-2-prop-2-ynyloxy-acetamide;
L) Antifungal biocontrol agents, plant bioactivators: *Ampelomyces quisqualis* (e.g. AQ 10^{®} from Intrachem Bio GmbH & Co. KG, Germany), *Aspergillus flavus* (e.g. AFLAGUARD^{®} from Syngenta, CH), *Aureobasidium pullulans* (e.g. BOTECTOR^{®} from bio-ferm GmbH, Germany), *Bacillus pumilus* (e.g. NRRL Accession No. B-30087 in SONATA^{®} and BALLAD^{®} Plus from AgraQuest Inc., USA), *Bacillus subtilis* (e.g. isolate NRRL-Nr. B-21661 in RHAPSODY^{®}, SERENADE^{®} MAX and SERENADE^{®} ASO from AgraQuest Inc., USA), *Bacillus subtilis* var. *amyloliquefaciens* FZB24 (e.g. TAEGRO^{®} from Novozyme Biologicals, Inc., USA), *Candida oleophila* I-82 (e.g. ASPIRE^{®} from Ecogen Inc., USA), *Candida saitoana* (e.g. BIOCURE^{®} (in mixture with lysozyme) and BIOCOAT^{®} from Micro Flo Company, USA (BASF SE) and Arysta), Chitosan (e.g. ARMOUR-ZEN from BotriZen Ltd., NZ), *Clonostachys rosea* f. *catenulata,* also named *Gliocladium catenulatum* (e.g. isolate J1446: PRESTOP^{®} from Verdera, Finland), *Coniothyrium minitans* (e.g. CONTANS^{®} from Prophyta, Germany), *Cryphonectria parasitica* (e.g. *Endothia parasitica* from CNICM, France), *Cryptococcus albidus* (e.g. YIELD PLUS^{®} from Anchor BioTechnologies, South Africa), *Fusarium oxysporum* (e.g. BIOFOX^{®} from S.I.A.P.A., Italy, FUSACLEAN^{®} from Natural Plant Protection, France), *Metschnikowia fructicola* (e.g. SHEMER^{®} from Agrogreen, Israel), *Microdochium dimerum* (e.g. ANTIBOT^{®} from Agrauxine, France), *Phlebiopsis gigantea* (e.g. ROTSOP^{®} from Verdera, Finland), *Pseudozyma flocculosa* (e.g. SPORODEX^{®} from Plant Products Co. Ltd., Canada), *Pythium oligandrum* DV74 (e.g. POLYVERSUM^{®} from Remeslo SSRO, Biopreparaty, Czech Rep.), *Reynoutria sachlinensis* (e.g. REGALIA^{®} from Marrone Biolnnovations, USA), *Talaromyces flavus* V117b (e.g. PROTUS^{®} from Prophyta, Germany), *Trichoderma asperellum* SKT-1 (e.g. ECO-HOPE^{®} from Kumiai Chemical Industry Co., Ltd., Japan), *T. atroviride* LC52 (e.g. SENTINEL^{®} from Agrimm Technologies Ltd, NZ), *T. harzianum* T-22 (e.g. PLANTSHIELD^{®} der Firma Bio-Works Inc., USA), *T. harzianum* TH 35 (e.g. ROOT PRO^{®} from Mycontrol Ltd., Israel), *T. harzianum* T-39 (e.g. TRICHODEX^{®} and TRICHODERMA 2000^{®} from Mycontrol Ltd., Israel and Makhteshim Ltd., Israel), *T. harzianum* and *T. viride* (e.g. TRICHOPEL from Agrimm Technologies Ltd, NZ), *T. harzianum* ICC012 and *T. viride* ICC080 (e.g. REMEDIER^{®} WP from Isagro Ricerca, Italy), *T. polysporum* and *T. harzianum* (e.g. BINAB^{®} from BINAB Bio-Innovation AB, Sweden), *T. stromaticum (e.g.* TRICOVAB^{®} from C.E.P.L.A.C., Brazil), *T. virens* GL-21 (e.g. SOIL-GARD^{®} from Certis LLC, USA), *T. viride* (e.g. TRIECO^{®} from Ecosense Labs. (India) Pvt. Ltd., Indien, BIO-CURE^{®} F from T. Stanes & Co. Ltd., Indien), *T. viride* TV1 (e.g. T. viride TV1 from Agribiotec srl, Italy), *Ulocladium oudemansii* HRU3 (e.g. BOTRY-ZEN^{®} from Botry-Zen Ltd, NZ);
M) Growth regulators
   abscisic acid, amidochlor, ancymidol, 6-benzylaminopurine, brassinolide, butralin, chlormequat (chlormequat chloride), choline chloride, cyclanilide, daminozide, dikegulac, dimethipin, 2,6-dimethylpuridine, ethephon, flumetralin, flurprimidol, fluthiacet, forchlorfenuron, gibberellic acid, inabenfide, indole-3-acetic acid , maleic hydrazide, mefluidide, mepiquat (mepiquat chloride), naphthaleneacetic acid, N-6-benzyladenine, paclobutrazol, prohexadione (prohexadione-calcium), prohydrojasmon, thidiazuron, triapenthenol, tributyl phosphorotrithioate, 2,3,5-tri-iodobenzoic acid , trinexapac-ethyl and uniconazole;
N) Herbicides
   - acetamides: acetochlor, alachlor, butachlor, dimethachlor, dimethenamid, flufenacet, mefenacet, metolachlor, metazachlor, napropamide, naproanilide, pethoxamid, pretilachlor, propachlor, thenylchlor;
   - amino acid derivatives: bilanafos, glyphosate, glufosinate, sulfosate;
   - aryloxyphenoxypropionates: clodinafop, cyhalofop-butyl, fenoxaprop, fluazifop, haloxyfop, metamifop, propaquizafop, quizalofop, quizalofop-P-tefuryl;
   - Bipyridyls: diquat, paraquat;
   - (thio)carbamates: asulam, butylate, carbetamide, desmedipham, dimepiperate, eptam (EPTC), esprocarb, molinate, orbencarb, phenmedipham, prosulfocarb, pyributicarb, thiobencarb, triallate;
   - cyclohexanediones: butroxydim, clethodim, cycloxydim, profoxydim, sethoxydim, tepraloxydim, tralkoxydim;
   - dinitroanilines: benfluralin, ethalfluralin, oryzalin, pendimethalin, prodiamine, trifluralin;
   - diphenyl ethers: acifluorfen, aclonifen, bifenox, diclofop, ethoxyfen, fomesafen, lactofen, oxyfluorfen;
   - hydroxybenzonitriles: bomoxynil, dichlobenil, ioxynil;
   - imidazolinones: imazamethabenz, imazamox, imazapic, imazapyr, imazaquin, imazethapyr;
   - phenoxy acetic acids: clomeprop, 2,4-dichlorophenoxyacetic acid (2,4-D), 2,4-DB, dichlorprop, MCPA, MCPA-thioethyl, MCPB, Mecoprop;
   - pyrazines: chloridazon, flufenpyr-ethyl, fluthiacet, norflurazon, pyridate;
   - pyridines: aminopyralid, clopyralid, diflufenican, dithiopyr, fluridone, fluroxypyr, picloram, picolinafen, thiazopyr;
   - sulfonyl ureas: amidosulfuron, azimsulfuron, bensulfuron, chlorimuron-ethyl, chlorsulfuron, cinosulfuron, cyclosulfamuron, ethoxysulfuron, flazasulfuron, flucetosulfuron, flupyrsulfuron, foramsulfuron, halosulfuron, imazosulfuron, iodosulfuron, mesosulfuron, metazosulfuron, metsulfuron-methyl, nicosulfuron, oxasulfuron, primisulfuron, prosulfuron, pyrazosulfuron, rimsulfuron, sulfometuron, sulfosulfuron, thifensulfuron, triasulfuron, tribenuron, trifloxysulfuron, triflusulfuron, tritosulfuron, 1-((2-chloro-6-propyl-imidazo[1,2-b]pyridazin-3-yl)sulfonyl)-3-(4,6-dimethoxy-pyrimidin-2-yl)urea;
   - triazines: ametryn, atrazine, cyanazine, dimethametryn, ethiozin, hexazinone, metamitron, metribuzin, prometryn, simazine, terbuthylazine, terbutryn, triaziflam;
   - ureas: chlorotoluron, daimuron, diuron, fluometuron, isoproturon, linuron, methabenzthiazuron,tebuthiuron;
   - other acetolactate synthase inhibitors: bispyribac-sodium, cloransulam-methyl, diclosulam, florasulam, flucarbazone, flumetsulam, metosulam, ortho-sulfamuron, penoxsulam, propoxycarbazone, pyribambenz-propyl, pyribenzoxim, pyriftalid, pyriminobac-methyl, pyrimisulfan, pyrithiobac, pyroxasulfone, pyroxsulam;
   - others: amicarbazone, aminotriazole, anilofos, beflubutamid, benazolin, bencarbazone,benfluresate, benzofenap, bentazone, benzobicyclon, bicyclopyrone, bromacil, bromobutide, butafenacil, butamifos, cafenstrole, carfentrazone, cinidon-ethyl, chlorthal, cinmethylin, clomazone, cumyluron, cyprosulfamide, dicamba, difenzoquat, diflufenzopyr, *Drechslera monoceras,* endothal, ethofumesate, etobenzanid, fenoxasulfone, fentrazamide, flumiclorac-pentyl, flumioxazin, flupoxam, flurochloridone, flurtamone, indanofan, isoxaben, isoxaflutole, lenacil, propanil, propyzamide, quinclorac, quinmerac, mesotrione, methyl arsonic acid, naptalam, oxadiargyl, oxadiazon, oxaziclomefone, pentoxazone, pinoxaden, pyraclonil, pyraflufen-ethyl, pyrasulfotole, pyrazoxyfen, pyrazolynate, quinoclamine, saflufenacil, sulcotrione, sulfentrazone, terbacil, tefuryltrione, tembotrione, thiencarbazone, topramezone, (3-[2-chloro-4-fluoro-5-(3-methyl-2,6-dioxo-4-trifluoromethyl-3,6-dihydro-2H-pyrimidin-1-yl)-phenoxy]-pyridin-2-yloxy)-acetic acid ethyl ester, 6-amino-5-chloro-2-cyclopropyl-pyrimidine-4-carboxylic acid methyl ester, 6-chloro-3-(2-cyclopropyl-6-methyl-phenoxy)-pyridazin-4-ol, 4-amino-3-chloro-6-(4-chloro-phenyl)-5-fluoro-pyridine-2-carboxylic acid, 4-amino-3-chloro-6-(4-chloro-2-fluoro-3-methoxy-phenyl)-pyridine-2-carboxylic acid methyl ester, and 4-amino-3-chloro-6-(4-chloro-3-dimethylamino-2-fluoro-phenyl)-pyridine-2-carboxylic acid methyl ester.
O) Insecticides
   - organo(thio)phosphates: acephate, azamethiphos, azinphos-methyl, chlorpyrifos, chlorpyrifos-methyl, chlorfenvinphos, diazinon, dichlorvos, dicrotophos, dimethoate, disulfoton, ethion, fenitrothion, fenthion, isoxathion, malathion, methamidophos, methidathion, methyl-parathion, mevinphos, monocrotophos, oxydemeton-methyl, paraoxon, parathion, phenthoate, phosalone, phosmet, phosphamidon, phorate, phoxim, pirimiphos-methyl, profenofos, prothiofos, sulprophos, tetrachlorvinphos, terbufos, triazophos, trichlorfon;
   - carbamates: alanycarb, aldicarb, bendiocarb, benfuracarb, carbaryl, carbofuran, carbosulfan, fenoxycarb, furathiocarb, methiocarb, methomyl, oxamyl, pirimicarb, propoxur, thiodicarb, triazamate;
   - pyrethroids: allethrin, bifenthrin, cyfluthrin, cyhalothrin, cyphenothrin, cypermethrin, alpha-cypermethrin, beta-cypermethrin, zeta-cypermethrin, deltamethrin, esfen-valerate, etofenprox, fenpropathrin, fenvalerate, imiprothrin, lambda-cyhalothrin, permethrin, prallethrin, pyrethrin I and II, resmethrin, silafluofen, tau-fluvalinate, tefluthrin, tetramethrin, tralomethrin, transfluthrin, profluthrin, dimefluthrin;
   - insect growth regulators: a) chitin synthesis inhibitors: benzoylureas: chlorfluazuron, cyramazin, diflubenzuron, flucycloxuron, flufenoxuron, hexaflumuron, lufenuron, novaluron, teflubenzuron, triflumuron; buprofezin, diofenolan, hexythiazox, etoxazole, clofentazine; b) ecdysone antagonists: halofenozide, methoxyfenozide, tebufenozide, azadirachtin; c) juvenoids: pyriproxyfen, methoprene, fenoxycarb; d) lipid biosynthesis inhibitors: spirodiclofen, spiromesifen, spirotetramat;
   - nicotinic receptor agonists/antagonists compounds: clothianidin, dinotefuran, flupyradifurone, imidacloprid, thiamethoxam, nitenpyram, acetamiprid, thiacloprid, 1-2-chloro-thiazol-5-ylmethyl)-2-nitrimino-3,5-dimethyl-[1,3,5]triazinane;
   - GABA antagonist compounds: endosulfan, ethiprole, fipronil, vaniliprole, pyrafluprole, pyriprole, 5-amino-1-(2,6-dichloro-4-methyl-phenyl)-4-sulfinamoyl-1 H-pyrazole-3-carbothioic acid amide;
   - macrocyclic lactone insecticides: abamectin, emamectin, milbemectin, lepimectin, spinosad, spinetoram;
   - mitochondrial electron transport inhibitor (METI) I acaricides: fenazaquin, pyridaben, tebufenpyrad, tolfenpyrad, flufenerim;
   - METI II and III compounds: acequinocyl, fluacyprim, hydramethylnon;
   - Uncouplers: chlorfenapyr;
   - oxidative phosphorylation inhibitors: cyhexatin, diafenthiuron, fenbutatin oxide, propargite;
   - moulting disruptor compounds: cryomazine;
   - mixed function oxidase inhibitors: piperonyl butoxide;
   - sodium channel blockers: indoxacarb, metaflumizone;
   - others: benclothiaz, bifenazate, cartap, flonicamid, pyridalyl, pymetrozine, sulfur, thiocyclam, flubendiamide, chlorantraniliprole, cyazypyr (HGW86), cyenopyrafen, flupyrazofos, cyflumetofen, amidoflumet, imicyafos, bistrifluron, and pyrifluquinazon.

The present invention furthermore relates to agrochemical compositions comprising a mixture of at least the co-crystals according to the invention (component 1) and at least one further active substance useful for plant protection, e. g. selected from the groups A) to O) (component 2), in particular one further fungicide, e. g. one or more fungicide from the groups A) to L), as described above, and if desired one suitable solvent or solid carrier. Those mixtures are of particular interest, since many of them at the same application rate show higher efficiencies against harmful fungi. Furthermore, combating harmful fungi with a mixture of the co-crystals and at least one fungicide from groups A) to L), as described above, is more efficient than combating those fungi with the co-crystalsalone or individual fungicides from groups A) to L). By applying the co-crystals together with at least one active substance from groups A) to O) a synergistic effect can be obtained, i.e. more then simple addition of the individual effects is obtained (synergistic mixtures).

This can be obtained by applying the co-crystals and at least one further active substance simultaneously, either jointly (e. g. as tank-mix) or seperately, or in succession, wherein the time interval between the individual applications is selected to ensure that the active substance applied first still occurs at the site of action in a sufficient amount at the time of application of the further active substance(s). The order of application is not essential for working of the present invention.

In ternary mixtures, i.e. compositions according to the invention comprising the co-crystals at least comprising cyprodinil and dithianon (component 1) and one further active substance (component 2), e. g. one active substance from groups A) to O), the weight ratio of component 1 and component 2 generally depends from the properties of the active substances used, usually it is in the range of from 1:100 to 100:1, regularly in the range of from 1:50 to 50:1, preferably in the range of from 1:20 to 20:1, more preferably in the range of from 1:10 to 10:1 and in particular in the range of from 1:3 to 3:1.

In quarternary mixtures, i.e. compositions according to the invention comprising the co-crystals at least comprising cyprodinil and dithianon (component 1) and a first further active substance (component 2) and a second further active substance (component 3), e. g. two active substances from groups A) to O), the weight ratio of component 1 and component 2 depends from the properties of the active substances used, preferably it is in the range of from 1:50 to 50:1 and particularly in the range of from 1:10 to 10:1, and the weight ratio of component 1 and component 3 preferably is in the range of from 1:50 to 50:1 and particularly in the range of from 1:10 to 10:1.

Preference is also given to mixtures comprising comprising the co-crystals (component 1) and at least one active substance selected from group A) (component 2) and particularly selected from azoxystrobin, dimoxystrobin, fluoxastrobin, kresoxim-methyl, orysastrobin, picoxystrobin, pyraclostrobin, trifloxystrobin; famoxadone, fenamidone; bixafen, boscalid, fluopyram, fluxapyroxad, isopyrazam, penflufen, penthiopyrad, sedaxane; ametoctradin, cyazofamid, fluazinam, fentin salts, such as fentin acetate.

Preference is given to mixtures comprising the co-crystals (component 1) and at least one active substance selected from group B) (component 2) and particularly selected from cyproconazole, difenoconazole, epoxiconazole, fluquinconazole, flusilazole, flutriafol, metconazole, myclobutanil, penconazole, propiconazole, prothioconazole, triadimefon, triadimenol, tebuconazole, tetraconazole, triticonazole, prochloraz, fenarimol, triforine; dodemorph, fenpropimorph, tridemorph, fenpropidin, spiroxamine; fenhexamid.

Preference is given to mixtures comprising the co-crystals (component 1) and at least one active substance selected from group C) (component 2) and particularly selected from metalaxyl, (metalaxyl-M) mefenoxam, ofurace.

Preference is given to mixtures comprising the co-crystals (component 1) and at least one active substance selected from group D) (component 2) and particularly selected from benomyl, carbendazim, thiophanate-methyl, ethaboxam, fluopicolide, zoxamide, metrafenone, pyriofenone.

Preference is also given to mixtures comprising the co-crystals (component 1) and at least one active substance selected from group E) (component 2) and particularly selected from cyprodinil, mepanipyrim, pyrimethanil.

Preference is also given to mixtures comprising the co-crystals (component 1) and at least one active substance selected from group F) (component 2) and particularly selected from iprodione, fludioxonil, vinclozolin, quinoxyfen.

Preference is also given to mixtures comprising the co-crystals (component 1) and at least one active substance selected from group G) (component 2) and particularly selected from dimethomorph, flumorph, iprovalicarb, benthiavalicarb, mandipropamid, propamocarb.

Preference is also given to mixtures comprising the co-crystals (component 1) and at least one active substance selected from group H) (component 2) and particularly selected from copper acetate, copper hydroxide, copper oxychloride, copper sulfate, sulfur, mancozeb, metiram, propineb, thiram, captafol, folpet, chlorothalonil, dichlofluanid, dithianon.

Preference is also given to mixtures comprising the co-crystals (component 1) and at least one active substance selected from group I) (component 2) and particularly selected from carpropamid and fenoxanil.

Preference is also given to mixtures comprising the co-crystals (component 1) and at least one active substance selected from group J) (component 2) and particularly selected from acibenzolar-S-methyl, probenazole, tiadinil, fosetyl, fosetyl-aluminium, H₃PO₃ and salts thereof.

Preference is also given to mixtures comprising the co-crystals (component 1) and at least one active substance selected from group K) (component 2) and particularly selected from cymoxanil, proquinazid and *N-*methyl-2-{1-[(5-methyl-3-trifluoromethyl-1 H-pyrazol-1-yl)-acetyl]-piperidin-4-yl}-*N*-[(1R)-1,2,3,4-tetrahydronaphthalen-1-yl]-4-thiazolecarboxamide.

Preference is also given to mixtures comprising the co-crystals (component 1) and at least one active substance selected from group L) (component 2) and particularly selected from *Bacillus subtilis* strain NRRL No. B-21661, *Bacillus pumilus* strain NRRL No. B-30087 and *Ulocladium oudemansii.*

The co-crystals and the compositions according to the invention, respectively, are suitable as fungicides. They are distinguished by an outstanding effectiveness against a broad spectrum of phytopathogenic fungi, including soil-borne fungi, which derive especially from the classes of the Plasmodiophoromycetes, Peronosporomycetes (syn. Oomycetes), Chytridiomycetes, Zygomycetes, Ascomycetes, Basidiomycetes and Deuteromycetes (syn. Fungi imperfecti). Some are systemically effective and they can be used in crop protection as foliar fungicides, fungicides for seed dressing and soil fungicides. Moreover, they are suitable for controlling harmful fungi, which inter alia occur in wood or roots of plants.

The co-crystals and the compositions according to the invention are particularly important in the control of a multitude of phytopathogenic fungi on various cultivated plants, such as cereals, e. g. wheat, rye, barley, triticale, oats or rice; beet, e. g. sugar beet or fodder beet; fruits, such as pomes, stone fruits or soft fruits, e. g. apples, pears, plums, peaches, almonds, cherries, strawberries, raspberries, blackberries or gooseberries; leguminous plants, such as lentils, peas, alfalfa or soybeans; oil plants, such as rape, mustard, olives, sunflowers, coconut, cocoa beans, castor oil plants, oil palms, ground nuts or soybeans; cucurbits, such as squashes, cucumber or melons; fiber plants, such as cotton, flax, hemp or jute; citrus fruit, such as oranges, lemons, grape-fruits or mandarins; vegetables, such as spinach, lettuce, asparagus, cabbages, carrots, onions, tomatoes, potatoes, cucurbits or paprika; lauraceous plants, such as avocados, cinnamon or camphor; energy and raw material plants, such as corn, soybean, rape, sugar cane or oil palm; corn; tobacco; nuts; coffee; tea; bananas; vines (table grapes and grape juice grape vines); hop; turf; sweet leaf (also called Stevia); natural rubber plants or ornamental and forestry plants, such as flowers, shrubs, broad-leaved trees or evergreens, e. g. conifers; and on the plant propagation material, such as seeds, and the crop material of these plants.

Preferably, the co-crystals and compositions thereof, respectively are used for controlling a multitude of fungi on field crops, such as potatoes sugar beets, tobacco, wheat, rye, barley, oats, rice, corn, cotton, soybeans, rape, legumes, sunflowers, coffee or sugar cane; fruits; vines; ornamentals; or vegetables, such as cucumbers, tomatoes, beans or squashes.

The term "plant propagation material" is to be understood to denote all the generative parts of the plant such as seeds and vegetative plant material such as cuttings and tubers (e. g. potatoes), which can be used for the multiplication of the plant. This includes seeds, roots, fruits, tubers, bulbs, rhizomes, shoots, sprouts and other parts of plants, including seedlings and young plants, which are to be transplanted after germination or after emergence from soil. These young plants may also be protected before transplantation by a total or partial treatment by immersion or pouring.

Preferably, treatment of plant propagation materials with the co-crystals and compositions thereof, respectively, is used for controlling a multitude of fungi on cereals, such as wheat, rye, barley and oats; rice, corn, cotton and soybeans.

The term "cultivated plants" is to be understood as including plants which have been modified by breeding, mutagenesis or genetic engineering including but not limiting to agricultural biotech products on the market or in development (cf. http://www.bio.org/speeches/pubs/er/agri_products.asp). Genetically modified plants are plants, which genetic material has been so modified by the use of recombinant DNA techniques that under natural circumstances cannot readily be obtained by cross breeding, mutations or natural recombination. Typically, one or more genes have been integrated into the genetic material of a genetically modified plant in order to improve certain properties of the plant. Such genetic modifications also include but are not limited to targeted post-translational modification of protein(s), oligo- or polypeptides e. g. by glycosylation or polymer additions such as prenylated, acetylated or farnesylated moieties or PEG moieties.

The co-crystals and compositions thereof, respectively, are particularly suitable for controlling the following plant diseases:
*Albugo* spp. (white rust) on ornamentals, vegetables (e. g. *A. candida*) and sunflowers (e. g. *A*. *tragopogonis*); *Alternaria* spp. (Alternaria leaf spot) on vegetables, rape (*A*. *brassicola* or *brassicae*)*,* sugar beets (*A*. *tenuis*), fruits, rice, soybeans, potatoes (e. g. *A. solani* or *A*. *alternata*), tomatoes (e. g. *A*. *solani* or *A*. *alternata*) and wheat; *Aphano-myces* spp. on sugar beets and vegetables; *Ascochyta* spp. on cereals and vegetables, e. g. *A*. *tritici* (anthracnose) on wheat and *A*. *hordei* on barley; *Bipolaris* and *Drechslera* spp. (teleomorph: *Cochliobolus* spp.), e. g. Southern leaf blight (*D. maydis*) or Northern leaf blight (*B. zeicola*) on corn, e. g. spot blotch (*B. sorokiniana*) on cereals and e.g. *B*. *oryzae* on rice and turfs; *Blumeria* (formerly *Erysiphe*) *graminis* (powdery mildew) on cereals (e. g. on wheat or barley); *Botrytis cinerea* (teleomorph: *Botryotinia fuckeliana:* grey mold) on fruits and berries (e. g. strawberries), vegetables (e. g. lettuce, carrots, celery and cabbages), rape, flowers, vines, forestry plants and wheat; *Bremia lactucae* (downy mildew) on lettuce; *Ceratocystis* (syn. *Ophiostoma*) spp. (rot or wilt) on broad-leaved trees and evergreens, e. g. *C. ulmi* (Dutch elm disease) on elms; *Cercospora* spp. (Cercospora leaf spots) on corn (e.g. Gray leaf spot: *C. zeae-maydis*), rice, sugar beets (e. g. *C. beticola*), sugar cane, vegetables, coffee, soybeans (e. g. *C. sojina* or *C*. *kikuchii*) and rice; *Cladosporium* spp. on tomatoes (e. g. *C. fulvum*: leaf mold) and cereals, e. g. *C. herbarum* (black ear) on wheat; *Claviceps purpurea* (ergot) on cereals; *Cochliobolus* (anamorph: *Helminthosporium* of *Bipolaris*) spp. (leaf spots) on corn (*C*. *carbonum*), cereals (e. g. *C. sativus,* anamorph: *B. sorokiniana*) and rice (e. g. *C*. *miyabeanus,* anamorph: *H. oryzae*); *Colletotrichum* (teleomorph: *Glomerella*) spp. (anthracnose) on cotton (e. g. *C. gossypii*), corn (e. g. *C. graminicola:* Anthracnose stalk rot), soft fruits, potatoes (e. g. *C. coccodes:* black dot), beans (e. g. *C. lindemuthianum*) and soybeans (e. g. *C. truncatum* or *C. gloeosporioides*); *Corticium* spp., e. g. *C. sasakii* (sheath blight) on rice; *Corynespora cassiicola* (leaf spots) on soybeans and ornamentals; *Cycloconium* spp., e. g. *C. oleaginum* on olive trees; *Cylindrocarpon* spp. (e. g. fruit tree canker or young vine decline, teleomorph: *Nectria* or *Neonectria* spp.) on fruit trees, vines (e. g. *C. liriodendri,* teleomorph: *Neonectria liriodendri:* Black Foot Disease) and ornamentals; *Dematophora* (teleomorph: *Rosellinia*) necatrix (root and stem rot) on soybeans; *Diaporthe* spp., e. g. *D. phaseolorum* (damping off) on soybeans; *Drechslera* (syn. *Helminthosporium,* teleomorph: *Pyrenophora*) spp. on corn, cereals, such as barley (e. g. *D. teres,* net blotch) and wheat (e. g. *D*. *tritici-repentis:* tan spot), rice and turf; Esca (dieback, apoplexy) on vines, caused by *Formitiporia* (syn. *Phellinus*) *punctata, F. mediterranea, Phaeomoniella chlamydospora* (earlier *Phaeoacremonium chlamydosporum*), *Phaeoacremonium aleophilum* and/or *Botryosphaeria obtusa; Elsinoe* spp. on pome fruits (*E. pyri*)*,* soft fruits (*E*. *veneta*: anthracnose) and vines (*E*. *ampelina:* anthracnose); *Entyloma oryzae* (leaf smut) on rice; *Epicoccum* spp. (black mold) on wheat; *Erysiphe* spp. (powdery mildew) on sugar beets (*E*. *betae*), vegetables (e. g. *E*. *pisi*), such as cucurbits (e. g. *E. cichoracearum*), cabbages, rape (e. g. *E*. *cruciferarum*); *Eutypa lata* (Eutypa canker or dieback, anamorph: *Cytosporina lata,* syn. *Libertella blepharis*) on fruit trees, vines and ornamental woods; *Exserohilum* (syn. *Helminthosporium*) spp. on corn (e. g. *E. turcicum*); *Fusarium* (teleomorph: *Gibberella*) spp. (wilt, root or stem rot) on various plants, such as *F. graminearum* or *F*. *culmorum* (root rot, scab or head blight) on cereals (e. g. wheat or barley), *F. oxysporum* on tomatoes, *F. solani* on soybeans and *F. verticillioides* on corn; *Gaeumannomyces graminis* (take-all) on cereals (e. g. wheat or barley) and corn; *Gibberella* spp. on cereals (e. g. *G. zeae*) and rice (e. g. *G. fujikuroi:* Bakanae disease); *Glomerella cingulata* on vines, pome fruits and other plants and *G. gossypii* on cotton; Grain-staining complex on rice; *Guignardia bidwellii* (black rot) on vines; *Gymnosporangium* spp. on rosaceous plants and junipers, e. g. *G. sabinae* (rust) on pears; *Helminthosporium* spp. (syn. *Drechslera,* teleomorph: *Cochliobolus*) on corn, cereals and rice; *Hemileia* spp., e. g. *H. vastatrix* (coffee leaf rust) on coffee; *Isariopsis clavispora* (syn. *Cladosporium vitis*) on vines; *Macrophomina phaseolina* (syn. *phaseoli*) (root and stem rot) on soybeans and cotton; *Microdochium* (syn. *Fusarium*) *nivale* (pink snow mold) on cereals (e. g. wheat or barley); *Microsphaera diffusa* (powdery mildew) on soybeans; *Monilinia* spp., e. g. *M. laxa, M. fructicola* and *M. fructigena* (bloom and twig blight, brown rot) on stone fruits and other rosaceous plants; *Mycosphaerella* spp. on cereals, bananas, soft fruits and ground nuts, such as e. g. *M. graminicola* (anamorph: *Septoria tritici,* Septoria blotch) on wheat or *M*. *fijiensis* (black Sigatoka disease) on bananas; *Peronospora* spp. (downy mildew) on cabbage (e. g. *P. brassicae*), rape (e. g. *P. parasitica*), onions (e. g. *P. destructor*), tobacco (*P*. *tabacina*) and soybeans (e. g. *P. manshurica*); *Phakopsora pachyrhizi* and *P. meibomiae* (soybean rust) on soybeans; *Phialophora* spp. e. g. on vines (e. g. *P. tracheiphila* and *P. tetraspora*) and soybeans (e. g. *P. gregata:* stem rot); *Phoma lingam* (root and stem rot) on rape and cabbage and *P*. *betae* (root rot, leaf spot and damping-off) on sugar beets; *Phomopsis* spp. on sunflowers, vines (e. g. *P. viticola:* can and leaf spot) and soybeans (e. g. stem rot: *P. phaseoli,* teleomorph: *Diaporthe phaseolorum*); *Physoderma maydis* (brown spots) on corn; *Phytophthora* spp. (wilt, root, leaf, fruit and stem root) on various plants, such as paprika and cucurbits (e. g. *P. capsici*), soybeans (e. g. *P. megasperma,* syn. *P. sojae*), potatoes and tomatoes (e. g. *P. infestans:* late blight) and broad-leaved trees (e. g. *P. ramorum:* sudden oak death); *Plasmodiophora brassicae* (club root) on cabbage, rape, radish and other plants; *Plasmopara* spp., e. g. *P*. *viticola* (grapevine downy mildew) on vines and *P. halstedii* on sunflowers; *Podosphaera* spp. (powdery mildew) on rosaceous plants, hop, pome and soft fruits, e. g. *P. leucotricha* on apples; *Polymyxa* spp., e. g. on cereals, such as barley and wheat (*P*. *graminis*) and sugar beets (*P. betae*) and thereby transmitted viral diseases; *Pseudocercosporella herpotrichoides* (eyespot, teleomorph: *Tapesia yallundae*) on cereals, e. g. wheat or barley; *Pseudoperonospora* (downy mildew) on various plants, e. g. *P*. *cubensis* on cucurbits or *P*. *humili* on hop; *Pseudopezicula tracheiphila* (red fire disease or, rotbrenner', anamorph: *Phialophora*) on vines; *Puccinia* spp. (rusts) on various plants, e. g. *P*. *triticina* (brown or leaf rust), *P*. *striiformis* (stripe or yellow rust), *P*. *hordei* (dwarf rust), *P*. *graminis* (stem or black rust) or *P*. *recondita* (brown or leaf rust) on cereals, such as e. g. wheat, barley or rye, *P*. *kuehnii* (orange rust) on sugar cane and *P*. *asparagi* on asparagus; *Pyrenophora* (anamorph: *Drechslera*) *tritici-repentis* (tan spot) on wheat or *P*. *teres* (net blotch) on barley; *Pyricularia* spp., e. g. *P*. *oryzae* (teleomorph: *Magnaporthe grisea,* rice blast) on rice and *P*. *grisea* on turf and cereals; *Pythium* spp. (damping-off) on turf, rice, corn, wheat, cotton, rape, sunflowers, soybeans, sugar beets, vegetables and various other plants (e. g. *P*. *ultimum* or *P*. *aphanidermatum*); *Ramularia* spp., e. g. *R. collo-cygni* (Ramularia leaf spots, Physiological leaf spots) on barley and *R. beticola* on sugar beets; *Rhizoctonia* spp. on cotton, rice, potatoes, turf, corn, rape, potatoes, sugar beets, vegetables and various other plants, e. g. *R. solani* (root and stem rot) on soybeans, *R. solani* (sheath blight) on rice or *R*. *cerealis* (Rhizoctonia spring blight) on wheat or barley; *Rhizopus stolonifer* (black mold, soft rot) on strawberries, carrots, cabbage, vines and tomatoes; *Rhynchosporium secalis* (scald) on barley, rye and triticale; *Sarocladium oryzae* and *S*. *attenuatum* (sheath rot) on rice; *Sclerotinia* spp. (stem rot or white mold) on vegetables and field crops, such as rape, sunflowers (e. g. *S*. *sclerotiorum*) and soybeans (e. g. *S*. *rolfsii* or *S*. *sclerotiorum*); *Septoria* spp. on various plants, e. g. *S*. *glycines* (brown spot) on soybeans, S. *tritici* (Septoria blotch) on wheat and S. (syn. *Stagonospora*) nodorum (Stagonospora blotch) on cereals; *Uncinula* (syn. *Erysiphe*) *necator* (powdery mildew, anamorph: *Oidium tuckeri*) on vines; *Setospaeria* spp. (leaf blight) on corn (e. g. *S*. *turcicum,* syn. *Helminthosporium turcicum*) and turf; *Sphacelotheca* spp. (smut) on corn, (e. g. *S*. *reiliana:* head smut), sorghum und sugar cane; *Sphaerotheca fuliginea* (powdery mildew) on cucurbits; *Spongospora subterranea* (powdery scab) on potatoes and thereby transmitted viral diseases; *Stagonospora* spp. on cereals, e. g. *S*. *nodorum* (Stagonospora blotch, teleomorph: *Leptosphaeria* [syn. *Phaeosphaeria*] *nodorum*) on wheat; *Synchytrium endobioticum* on potatoes (potato wart disease); *Taphrina* spp., e. g. *T. deformans* (leaf curl disease) on peaches and *T. pruni* (plum pocket) on plums; *Thielaviopsis* spp. (black root rot) on tobacco, pome fruits, vegetables, soybeans and cotton, e. g. *T. basicola* (syn. *Chalara elegans*); *Tilletia* spp. (common bunt or stinking smut) on cereals, such as e. g. *T. tritici* (syn. *T. caries,* wheat bunt) and *T. controversa* (dwarf bunt) on wheat; *Typhula incarnata* (grey snow mold) on barley or wheat; *Urocystis* spp., e. g. *U. occulta* (stem smut) on rye; *Uromyces* spp. (rust) on vegetables, such as beans (e. g. *U. appendiculatus,* syn. *U. phaseoli*) and sugar beets (e. g. *U. betae*); *Ustilago* spp. (loose smut) on cereals (e. g. *U. nuda* and *U. avaenae*), corn (e. g. *U. maydis:* corn smut) and sugar cane; *Venturia* spp. (scab) on apples (e. g. *V. inaequalis*) and pears; and *Verticillium* spp. (wilt) on various plants, such as fruits and ornamentals, vines, soft fruits, vegetables and field crops, e. g. *V. dahliae* on strawberries, rape, potatoes and tomatoes.

The co-crystals and compositions thereof, respectively, are also suitable for controlling harmful fungi in the protection of stored products or harvest and in the protection of materials. The term "protection of materials" is to be understood to denote the protection of technical and non-living materials, such as adhesives, glues, wood, paper and paperboard, textiles, leather, paint dispersions, plastics, colling lubricants, fiber or fabrics, against the infestation and destruction by harmful microorganisms, such as fungi and bacteria. As to the protection of wood and other materials, the particular attention is paid to the following harmful fungi: Ascomycetes such as *Ophiostoma* spp., *Ceratocystis* spp., *Aureobasidium pullulans, Sclerophoma* spp., *Chaetomium* spp., *Humicola* spp., *Petriella* spp., *Trichurus* spp.; Basidiomycetes such as *Coniophora* spp., *Coriolus* spp., *Gloeophyllum* spp., *Lentinus* spp., *Pleurotus* spp., *Poria* spp., *Serpula* spp. and *Tyromyces* spp., Deuteromycetes such as *Aspergillus* spp., *Cladosporium* spp., *Penicillium* spp., *Trichorma* spp., *Alternaria* spp., *Paecilomyces* spp. and Zygomycetes such as *Mucor* spp., and in addition in the protection of stored products and harvest the following yeast fungi are worthy of note: *Candida* spp. and *Saccharomyces cerevisae.*

The co-crystals and compositions thereof, resepectively, may be used for improving the health of a plant. The invention also relates to a method for improving plant health by treating a plant, its propagation material and/or the locus where the plant is growing or is to grow with an effective amount of the co-crystals and compositions thereof, respectively.

The term "plant health" is to be understood to denote a condition of the plant and/or its products which is determined by several indicators alone or in combination with each other such as yield (e. g. increased biomass and/or increased content of valuable ingredients), plant vigor (e. g. improved plant growth and/or greener leaves (" greening effect" )), quality (e. g. improved content or composition of certain ingredients) and tolerance to abiotic and/or biotic stress.The above identified indicators for the health condition of a plant may be interdependent or may result from each other.

The co-crystals are employed as such or in form of compositions by treating the fungi or the plants, plant propagation materials, such as seeds, soil, surfaces, materials or rooms to be protected from fungal attack with a fungicidally effective amount of the active substances. The application can be carried out both before and after the infection of the plants, plant propagation materials, such as seeds, soil, surfaces, materials or rooms by the fungi. Plant propagation materials may be treated with the co-crystals as such or a composition comprising at least one compound I prophylactically either at or before planting or transplanting.

### Analysis:

### X-ray powder diffractometry (XRPD):

The X-ray powder diffractograms (XRPD) used to identify the different solid modifications (the co-crystal of cyprodinil and dithianon, dithianon of Form I and cyprodinil of Form A) were recorded using a Panalytical X'Pert Pro diffractometer (manufacturer: Panalytical) in reflection geometry in the range from 2θ =3°-35°C with increments of 0,0167°C using Cu-Kα radiation at 20°C. A monocromator was not applied. A zero background sample holder and a sample amount of 10 to 30 mg was used in each measurement. The recorded 2θ values were used to calculate the stated interplanar spacings d. The characteristic diffraction lines are given in Table 1. The intensity of the peaks (y-axis: linear intensity counts) is plotted ver sus the 2θ angle (x-axis in degrees 2θ) in Figure 1.

### Thermal analysis of the co-crystal

DSC-measurement was carried out with a Mettler Toledo DSC 25 instrument. An open aluminium pan was used and the measurement was carried out under nitrogen flow with a heating rate of 5°C/min and a sample weight of 5 to 10 mg.

TG/DTA measurement was carried out on a Seiko TG/DTA 7200 instrument. An open aluminium pan was used and the measurement was carried out under nitrogen flow with a heating rate of 5oC/min and a sample weight of 5 to 10 mg.

The Examples and Figures below serve to illustrate the invention and are not to be understood as limiting it.
- Figure 1:: Powder X-ray diffractogram of the co-crystals of cyprodinil and dithianon.
- Figure 2:: Differential Scanning Calorimetry-trace of the co-crystals of cyprodinil and dithianon.
- Figure 3:: TG/DTA trace of the co-crystals of cyprodinil and dithianon.

(Only co-crystals falling under the scope of the claims form part of the invention).

### Example 1: Preparation of the co-crystal via solvent drop grinding

The co-crystal was prepared as follows. Dithianon (852 mg) and cyprodinil (645 mg), were mixed with 5 drops of ethanol in a small lab scale ball mill. The mixture was milled 30 minutes with a frequency of 20 Hz. The co-crystals were dark brown to blackish. The co-crystal was analyzed using PXRD, DSC and TG/DTA. The results are shown in Figures 1, 2 and 3.

### Example 2: Two separate premixes are prepared.

### Premix A:

A mixture of 35 g 1,2-Propylenglycol, 10 g of Soprophor BSU, 15 g Pluronic PE 10500, 5 g Atplus 245, 1 g of Acticide MBS and 125 g deionised water can be homogenized under stirring until a clear solution is obtained. 300 g Cyprodinil can be added to this solution. The resulting suspension can be milled over a glass-bead mill to obtain a particle size distribution (PSD) of about 60% < 2 μm.

### Premix B:

A mixture of 35 g 1,2-Propylenglycol, 10 g of Soprophor BSU, 15 g Pluronic PE 10500, 5 g Atplus 245, 1 g of Acticide MBS and 125 g deionised water can be homogenized under stirring until a clear solution is obtained. 300 g Dithianon can be added to this solution. The resulting suspension can be milled over a glass-bead mill to obtain a PSD of about 60% < 2 μm.

The two premixes A and B can be mixed together in one beaker under stirring at 50°C. After formation of the co-crystals within about 1 hour to maximally 12 hours, the suspension shall be cooled to 20°C and then milled over a glass-bead mill to obtain a PSD of about 60% < 2 μm.

### Example 3:

The premixes A and B can be passed continously over a bead-mill at elevated temperature. After formation of the co-crystals within about 1 hour to maximally 12 hours, the mill can be cooled to 20 °C and milling is extended until a PSD of about 60% < 2 μm is achieved.

### Example 4:

A mixture of 70 g 1,2-Propylenglycol, 20 g of Soprophor BSU, 30 g Pluronic PE 10500, 10 g Atplus 245, 2 g of Acticide MBS and 250 g deionised water can be homogenized under stirring until a clear solution is obtained. 300 g Cyprodinil can be added to this solution. The resulting suspension can be milled over a glass-bead mill to obtain a particle size distribution of 80 % < 2 μm. To this suspension premix can be added 300 g dithianon under agitation. Thereafter, the mixture can be heated to 50°C for at least 1 hour. After cooling it down to 20°C it can be milled over a bead mill to obtain a PSD of about 60% < 2 μm.

### Example 5:

A mixture of 70 g 1,2-Propylenglycol, 20 g of Soprophor BSU, 30 g Pluronic PE 10500, 10 g Atplus 245, 2 g of Acticide MBS and 250 g deionised water can be homogenized under stirring until a clear solution is obtained. 300 g Dithianon can be added to this solution. The resulting suspension can be milled over a glass-bead mill to obtain a PSD of about 80 % < 2 μm. To this suspension premix can be added 300 g cyprodinil under agitation. Thereafter, the mixture can be heated to 50°C for 1 hour. After cooling it down to 20°C it can be milled over a bead mill to obtain a PSD of about 60% < 2 μm.

### Example 6:

Dithianon and cyprodinil can be suspended together with suitable dispersants and emulsifiers in a mineral or vegetable based oil (like white oil, Bayol 85, sun flower oil, rape seed oil, corn oil, also methyloleate). The OD (oil dispersion) premix can be heated under stirring to 50°C for at least about 1 hour. Then the premix can be cooled down and grinded over a bead mill to obtain a PSD of about 60% < 2 μm.

### Example 7:

Solutions of 296,3 g dithianon and 199,4 cyprodinil in polar solvents like DMSO, Acetonitril, DMF or aceton can be prepared. The solutions are mixed and the respective solvent can be evaporated to yield the co-crystals. These can be suspended in water plus formulation additives (same as for suspension concentrate) and first pre-ground over a PUC mill and then finely ground over a bead mill to achieve a PSD of about 60% < 2 μm.

### Example 8:

A suspension premix of cyprodinil and dithianon can be prepared as described in example no. 4. This premix can then be run over a bead mill to give a PSD of about 60% < 2 μm. The resulting suspension can then be spray dried to give a WDG formulation.

### Example 9:

A mixture of 70 g 1,2-Propylenglycol, 20 g of Soprophor BSU, 30 g Pluronic PE 10500, 10 g Lutensol TO 65, 2 g of Acticide MBS and 250 g deionised water was homogenized under stirring until a clear solution was obtained. 254,3 g Cyprodinil TGAI (98,3%) and 255,4 g Dithianon TGAI (97,3%) were added to this solution. The resulting suspension was heated to 45° C under stirring for one hour. The suspension changed its color from brown to dark blue and after cooling it down to 20°C it was milled over a glass bead mill to obtain a PSD of 80 % < 2 μm. The resulting suspension has low viscosity and dispersed finely in water to yield an appropriate spay liquid for the application as plant protection agent.

### Comparison examples:

A mixture of 70 g 1,2-Propylenglycol, 20 g of Soprophor BSU, 30 g Pluronic PE 10500, 10 g Lutensol TO 65, 2 g of Acticide MBS and 250 g deionised water was homogenized under stirring until a clear solution is obtained. 254,3 g cyprodnil TGAI (technical grade active ingredient; purity 98,3 %) and 255,4 g Dithianon TGAI (purity 97,9%) were added to this solution. After a single pass over a colloid mill the resulting suspension was milled over a glass-bead mill to obtain a particle size distribution of 80 % < 2 μm. During the milling viscosity was found to increase rapidly and solidify within minutes. The formulation process had to be stopped, the resulting product could not be used.

In another experiment the same recipe was tried to grind with glass beads in a small cylindrical agitated tube (IKA tube drive). The content of the mill solidified as well. The resulting cylindrical bloc of formulation could not be suspended in water.

## Claims

1. Co-crystals comprising cyprodinil and dithianon , which, in a powder X-ray diffractogram at 20°C, show at least three of the following 2θ values [°]:
7.47±0.2; 9.86±0.2°; 12.34±0.2°; 12.88±0.2°; 13.92±0.2°; 14.88±0.2°; 15.34±0.2°; 16.20±0.2°; 17.85±0.2°; 18.97±0.2°; 19.69±0.2°; 20.04±0.2°; 24.44±0.2°; 26.11±0.2°; 27.97±0.2°.

2. The co-crystals according to claim1 having a melting point in the range of 125 to 145°.

3. The co-crystals according to any of the claims 1 to 2 consisting of about equimolar amounts of cyprodinil and dithianon.

4. A process for the preparation of the co-crystals as defined in any of the claims 1 to 3, comprising the following steps:
i) dry-mixing about equimolar amounts of cyprodinil and dithianon to form a premix,
ii) dry-grinding the premix to form the co-crystals.

5. A process for the preparation of the co-crystals as defined in any of the claims 1 to 3, comprising the following steps:
i) dissolving about equimolar amounts of cyprodinil and dithianon in a polar organic solvent,
ii) evaporating the solvent to form the co-crystals.

6. A process for the preparation of the co-crystals as defined in any of the claims 1 to 3, comprising the following steps:
i) mixing about equimolar amounts of cyprodinil and dithianon with a liquid carrier to form a premix,
ii) grinding or agitating the premix or applying shear forces to the premix to form the co-crystals in the liquid carrier.

7. A composition for crop protection, comprising co-crystals as defined in any of the claims 1 to 3 and at least one auxiliary.

8. The composition according to claim 7, further comprising cyprodinil and/or dithianon.

9. The composition according to any of the claim7 to 8, comprising at least one further active substance.

10. The composition according to any of the claims 7 to 9 in the form of a suspension concentrate.

11. The composition according to claim 10 in the form of an aqueous or vegetable oil or mineral oil based suspension concentrate.

12. A process for the preparation of an aqueous suspension concentrate as defined in any of the claims 10 to 11, comprising the following steps:
i) adding cyprodinil into water which contains one or more dispersing agents and optionally other auxiliaries to form a premix A,
ii) adding dithianon into water which contains one or more dispersing agents and optionally other auxiliaries to form a premix B, and mixing the premixes A and B together under stirring at a temperature from 20°C to 80°C to form a suspension of co-crystals as defined in any of the claims 1 to 4, or
mixing the premix A with dithianon under stirring at a temperature from 20°C to 80°C to form a suspension of co-crystals as defined in any of the claims 1 to 4,
iii) milling the so obtained suspension to a particle size distribution of 60 % < 2 μm.

13. A process for the preparation of an aqueous suspension concentrate as defined in any of the claims 10 to 11, comprising the following steps:
i) adding dithianon into water which contains one or more dispersing agents and optionally other auxiliaries to form a premix B,
ii) mixing the premix B with cyprodinil under stirring at a temperature from 20°C to 80°C to form a suspension of co-crystals as defined any of the claims 1 to 4,
iii) milling the so obtained suspension to a particle size distribution of 60 % < 2 μm.

14. Co-crystals as defined in any of the claims 1 to 3, or of a composition as defined in any of the claim 7 to 11 for use in combating phytopathogenic fungi.

## Patentansprüche

1. Co-Kristalle umfassend Cyprodinil und Dithianon, die im Röntgenpulverdiffraktogramm bei 20°C mindestens drei der folgenden 2θ-Werte [°] zeigen: 7,47±0,2; 9,86±0,2°; 12,34±0,2°; 12,88±0,2°; 13,92±0,2°; 14,88±0,2°; 15,34±0,2°; 16,20±0,2°; 17,85±0,2°; 18,97±0,2°; 19,69±0,2°; 20,04±0,2°; 24,44±0,2°; 26,11±0,2°; 27,97±0,2°.

2. Co-Kristalle nach Anspruch 1 mit einem Schmelzpunkt im Bereich von 125 bis 145°.

3. Co-Kristalle nach einem der Ansprüche 1 bis 2, die aus ungefähr äquimolaren Mengen an Cyprodinil und Dithianon bestehen.

4. Verfahren zur Herstellung der Co-Kristalle wie in einem der Ansprüche 1 bis 3 definiert, das die folgenden Schritte umfasst:
i) Trockenmischen von ungefähr äquimolaren Mengen an Cyprodinil und Dithianon, wodurch eine Vormischung gebildet wird,
ii) Trockenmahlen der Vormischung, wodurch die Co-Kristalle gebildet werden.

5. Verfahren zur Herstellung der Co-Kristalle wie in einem der Ansprüche 1 bis 3 definiert, das die folgenden Schritte umfasst:
i) Lösen von ungefähr äquimolaren Mengen an Cyprodinil und Dithianon in einem polaren organischen Lösungsmittel,
ii) Abdampfen des Lösungsmittels, wodurch die Co-Kristalle gebildet werden.

6. Verfahren zur Herstellung der Co-Kristalle wie in einem der Ansprüche 1 bis 3 definiert, das die folgenden Schritte umfasst:
i) Lösen von ungefähr äquimolaren Mengen an Cyprodinil und Dithianon mit einem flüssigen Träger, wodurch eine Vormischung gebildet wird,
ii) Mahlen oder Bewegen der Vormischung oder Einwirkenlassen von Scherkräften auf die Vormischung, wodurch die Co-Kristalle in dem flüssigen Träger gebildet werden.

7. Zusammensetzung für den Pflanzenschutz, die Co-Kristalle wie in einem der Ansprüche 1 bis 3 definiert und mindestens einen Hilfsstoff umfasst.

8. Zusammensetzung nach Anspruch 7, die weiterhin Cyprodinil und/oder Dithianon umfasst.

9. Zusammensetzung nach einem der Ansprüche 7 bis 8, die mindestens einen weiteren Wirkstoff umfasst.

10. Zusammensetzung nach einem der Ansprüche 7 bis 9 in Form eines Suspensionskonzentrats.

11. Zusammensetzung nach Anspruch 10 in Form eines wässrigen Suspensionskonzentrats oder eines Suspensionskonzentrats auf Pflanzen- oder Mineralölsbasis.

12. Verfahren zur Herstellung eines wässrigen Supensionskonzentrats wie in einem der Ansprüche 10 bis 11 definiert, das die folgenden Schritte umfasst:
i) Zugeben von Cyprodinil zu Wasser, das ein oder mehrere Dispergiermittel und gegebenenfalls weitere Hilfsstoffe umfasst, wodurch eine Vormischung A gebildet wird,
ii) Zugeben von Dithianon zu Wasser, das ein oder mehrere Dispergiermittel und gegebenenfalls weitere Hilfsstoffe umfasst, wodurch eine Vormischung B gebildet wird, und Zusammenmischen der Vormischungen A und B unter Rühren bei einer Temperatur von 20°C bis 80°C, wodurch eine Suspension von Co-Kristallen wie in einem der Ansprüche 1 bis 4 definiert gebildet wird, oder
Mischen der Vormischung A mit Dithianon unter Rühren bei einer Temperatur von 20°C bis 80°C, wodurch eine Suspension von Co-Kristallen wie in einem der Ansprüche 1 bis 4 definiert gebildet wird,
iii) Mahlen der so erhaltenen Suspension auf eine Teilchengrößenverteilung von 60% < 2 μm.

13. Verfahren zur Herstellung eines wässrigen Supensionskonzentrats wie in einem der Ansprüche 10 bis 11 definiert, das die folgenden Schritte umfasst:
i) Zugeben von Dithianon zu Wasser, das ein oder mehrere Dispergiermittel und gegebenenfalls weitere Hilfsstoffe umfasst, wodurch eine Vormischung B gebildet wird,
ii) Mischen der Vormischung B mit Cyprodinil unter Rühren bei einer Temperatur von 20°C bis 80°C, wodurch eine Suspension von Co-Kristallen wie in einem der Ansprüche 1 bis 4 definiert gebildet wird,
iii) Mahlen der so erhaltenen Suspension auf eine Teilchengrößenverteilung von 60% < 20 μm.

14. Co-Kristalle wie in einem der Ansprüche 1 bis 3 definiert oder einer Zusammensetzung wie in einem der Ansprüche 7 bis 11 definiert, zur Verwendung in der Bekämpfung von phytopathogenen Pilzen.

## Revendications

1. Co-cristaux comprenant du cyprodinil et de la dithianone qui, dans un diffractogramme de rayons X de poudre à 20 °C, présente au moins trois des valeurs de 2θ [°] suivantes :
7,47 ± 0,2 ; 9,86 ± 0,2° ; 12,34 ± 0,2° ; 12,88 ± 0,2° ; 13,92 ± 0,2°; 14,88 ± 0,2°; 15,34 ± 0,2°; 16,20 ± 0,2°; 17,85 ± 0,2°; 18,97 ± 0,2°; 19,69 ± 0,2°; 20,04 ± 0,2°; 24,44 ± 0,2°; 26,11 ± 0,2°; 27,97 ± 0,2°.

2. Co-cristaux selon la revendication 1 présentant un point de fusion dans l'intervalle allant de 125 à 145°.

3. Co-cristaux selon l'une quelconque des revendications 1 à 2 constitués de quantités environ équimolaires de cyprodinil et dithianone.

4. Procédé de préparation des co-cristaux selon l'une quelconque des revendications 1 à 3, comprenant les étapes suivantes :
i) mélangeage à sec de quantités environ équimolaires de cyprodinil et de dithianone pour former un prémélange,
ii) broyage à sec du prémélange pour former les co-cristaux.

5. Procédé de préparation des co-cristaux selon l'une quelconque des revendications 1 à 3, comprenant les étapes suivantes :
i) dissolution de quantités environ équimolaires de cyprodinil et de dithianone dans un solvant organique polaire,
ii) évaporation du solvant pour former les co-cristaux.

6. Procédé de préparation des co-cristaux selon l'une quelconque des revendications 1 à 3, comprenant les étapes suivantes :
i) mélangeage de quantités environ équimolaires de cyprodinil et de dithianone avec un vecteur liquide pour former un prémélange,
ii) broyage ou agitation du prémélange ou application de forces de cisaillement au prémélange pour former les co-cristaux dans le vecteur liquide.

7. Composition pour la protection des cultures, comprenant des co-cristaux selon l'une quelconque des revendications 1 à 3 et au moins un auxiliaire.

8. Composition selon la revendication 7, comprenant en outre du cyprodinil et/ou de la dithianone.

9. Composition selon l'une quelconque des revendications 7 à 8, comprenant au moins un principe actif supplémentaire.

10. Composition selon l'une quelconque des revendications 7 à 9 sous la forme d'une suspension concentrée.

11. Composition selon la revendication 10 sous la forme d'une suspension concentrée à base aqueuse ou d'huile végétale ou minérale.

12. Procédé de préparation d'une suspension concentrée aqueuse selon l'une quelconque des revendications 10 à 11, comprenant les étapes suivantes :
i) ajout de cyprodinil à de l'eau contenant un ou plusieurs agents de dispersion et éventuellement d'autres auxiliaires pour former un prémélange A,
ii) ajout de dithianone dans de l'eau qui contient un ou plusieurs agents de dispersion et éventuellement d'autres auxiliaires pour former un prémélange B, et mélangeage des prémélanges A et B sous agitation à une température comprise entre 20 °C et 80 °C pour former une suspension de co-cristaux selon l'une quelconque des revendications 1 à 4, ou
mélangeage du prémélange A avec la dithianone sous agitation à une température comprise entre 20 °C et 80 °C pour former une suspension de co-cristaux selon l'une quelconque des revendications 1 à 4,
iii) meulage de la suspension ainsi obtenue jusqu'à une distribution granulométrique de 60 % < 2 μm.

13. Procédé de préparation d'une suspension concentrée aqueuse selon l'une quelconque des revendications 10 à 11, comprenant les étapes suivantes :
i) ajout de dithianone à de l'eau contenant un ou plusieurs agents de dispersion et éventuellement d'autres auxiliaires pour former un prémélange B,
ii) mélangeage du prémélange B avec le cyprodinil sous agitation à une température comprise entre 20 °C et 80 °C pour former une suspension de co-cristaux selon l'une quelconque des revendications 1 à 4,
iii) meulage de la suspension ainsi obtenue jusqu'à une distribution granulométrique de 60 % < 2 μm.

14. Co-cristaux selon l'une quelconque des revendications 1 à 3, ou composition selon l'une quelconque des revendications 7 à 11 pour utilisation dans la lutte contre les champignons phytopathogènes.
